(19) **Europäisches Patentamt / European Patent Office / Office européen des brevets**

(11) **EP 1 341 466 B1**

(12) **EUROPEAN PATENT SPECIFICATION**

(45) Date of publication and mention
of the grant of the patent:
**23.09.2009 Bulletin 2009/39**

(21) Application number: **00980498.0**

(22) Date of filing: **17.11.2000**

(51) Int Cl.:
***A61B 19/00*** *(2006.01)*

(86) International application number:
**PCT/US2000/031673**

(87) International publication number:
**WO 2002/039918 (23.05.2002 Gazette 2002/21)**

(54) **SYSTEMS FOR STABILIZING A TARGET LOCATION WITHIN A HUMAN BODY**

SYSTEME ZUR STABILISIERUNG EINER ZIELPOSITION IN EINEM MENSCHLICHEN KÖRPER

SYSTEMES DE STABILISATION DE LA POSITION D'UNE CIBLE A L'INTERIEUR DU CORPS HUMAIN

(84) Designated Contracting States:
**AT BE CH CY DE DK ES FI FR GB GR IE IT LI LU MC NL PT SE TR**

(43) Date of publication of application:
**10.09.2003 Bulletin 2003/37**

(73) Proprietor: **Calypso Medical, Inc**
**Seattle, WA 98101 (US)**

(72) Inventors:
• **KRAG, David**
**Shelburne, VT 05482 (US)**

• **DIMMER, Steve**
**Bellevue, WA 98005 (US)**

(74) Representative: **Gowshall, Jonathan Vallance et al**
**Forrester & Boehmert**
**Pettenkoferstrasse 20-22**
**80336 München (DE)**

(56) References cited:
**EP-A- 0 531 081   WO-A-00/51514**
**WO-A-96/08208   WO-A-97/12553**
**WO-A-98/38908   WO-A-99/30182**
**WO-A-99/58065   US-A- 5 902 310**
**US-A- 6 053 937**

EP 1 341 466 B1

**Description**

TECHNICAL FIELD

Several aspects of the present invention relate to systems for locating a target tissue within a human body with wireless markers. Other aspects of the invention relate to wireless markers, and instruments.

BACKGROUND

Many medical procedures require monitoring or treating an internal tissue mass or other body part within a human body. In such applications, medical procedures must accurately locate a small target location within a soft tissue region, an organ, a bone structure, or another body part (*e.g.*, colon, vascular system, etc.). The small target location can be a lesion, polyp, tumor, or another area of interest for monitoring or treating a patient. One particular application involving the surgical treatment of cancer is particularly challenging because physicians often need to treat small, non-palpable lesions that cannot be observed. This problem is compounded in soft tissue applications because the soft tissue is mobile and can move with respect to a reference point on the patient. In the case of breast cancer, for example, the location of a non-palpable lesion in the breast is identified at a pre-operative stage using an imaging system. The actual surgical procedure, however, occurs in an operating room at a subsequent point in time, and the patient is typically in a different position during the surgical procedure than during the pre-operative imaging stage. The breast and the lesion may accordingly be in a different location relative to a reference point on the patient during the surgical procedure than the imaging stage. The physician, therefore, generally estimates the location of lesion during surgery.

One problem with treating non-palpable lesions in soft tissues is that the physicians may incorrectly estimate the location of the lesions. As a result, the physician may not remove all of the lesion, which is not desirable because some of the lesion will accordingly remain in the soft tissue. Another result is that the physicians may remove a significant amount of tissue proximate to the lesion, which can cause undesirable collateral damage to healthy tissue. Therefore, it would be desirable to know the precise location of the lesion or other type of target location during the surgical procedure.

A current technique for performing an excisional biopsy of a non-palpable breast lesion that has been identified by mammogram or other method involves placement of a needle or guide wire (*e.g.*, a "Kopanz wire"), with or without blue dye, to guide the surgeon to the lesion. The tip of the needle is generally placed directly in or as close as possible to the lesion. When larger or more complex lesions are encountered, two or more guide wires are sometimes placed at each edge of the lesion. The entry point of the needle through the skin of the breast is usually several centimeters from the lesion due to the logistics of needle placement. The surgeon does not cut along the shaft of the needle from the skin because the distance is too great. Instead, the surgeon must estimate where in the breast the lesion is located by making reference to the location of the needle.

This technique is not optimal because it can be difficult to properly define the margins of the tissue that is to be removed, both during and after insertion of the needle(s), in tissue that is amorphous and pliable (*e.g.*, breast tissue). Also, it is often difficult for the surgeon to detect the exact depth of the lesion based on the placement of the needles. For these reasons it is not uncommon that the biopsied tissue does not contain the mammographically positive specimen. In other cases, as a result of the difficulty of estimating the proper location of the boundaries of the volume of tissue to be removed, the lesion ends up being eccentrically positioned within the volume of tissue excised. This calls into question the adequacy of the margin of normal tissue surrounding the lesion. In still other cases, more normal tissue is removed than is required, which is disadvantageous in this era of tissue-conserving therapies.

In other fields of surgery it is known to target portions of a human body using various devices, and then refer to such devices in connection with the removal or treatment of such portions. For example, U.S. Patent No. 5,630,431 to Taylor (the "'431 patent") describes a surgical manipulator that is controlled, in part, by information received from beacons that are positioned proximate to a region of a human body to be treated. As another example, U.S. Patent No. 5,397,329 to Allen (the "'329 patent") describes fiducial implants for a human body that are detectable by an imaging system. The fiducial implants are implanted beneath the skin and are spaced sufficiently from one another to define a plane that is detectable by the imaging system and is used in connection with creation of images of a body portion of interest. These images are then used, for instance, in eliminating a tumor by laser beam.

Unfortunately, the devices described in the '431 and '329 patents are vastly more complex, and hence expensive, than is appropriate for many surgical procedures. This problem is particularly disadvantageous with the emphasis on containing costs in managed health care. Furthermore, due to the amorphous, pliable nature of certain tissue, the systems of the '431 and '329 patents cannot be used effectively. Systems of the type described in the '431 and '329 patents require that the devices (*e.g.*, beacons or fiducial implants) defining the body portions of interest be substantially fixed relative to one another and relative to such body portions. These systems generally function effectively when the devices defining the body portion of interest are inserted in bone, *e.g.*, in a skull in connection with brain surgery or treatment, but are not believed to operate as intended when the devices are inserted in amorphous, pliable tissue.

Breast lesions are typically excised with a scalpel manipulated directly by the surgeon. With the current emphasis on surgical therapies that conserve breast tissue, the above-described procedure for removing a breast lesion is typically performed through a narrow opening in the skin created by slitting and then pulling apart the skin. It tends to be difficult to manipulate the scalpel within this opening so as to remove the desired volume of tissue. The amorphous, pliable nature of breast tissue exacerbates removal of such tissue inasmuch as application of force to the scalpel causes movement of the breast tissue relative to the opening in the skin.

Circular cutting tools are not widely used in surgery. Recently, however, United States Surgical Corporation of Norwalk, Connecticut, introduced a relatively small diameter, *e.g.*, 5-20 mm, circular cutting tool identified by the trademark ABBI for removing a cylinder of breast tissue for biopsy purposes. The ABBI tool includes an oscillating, motorized, circular cutting blade that incises the breast tissue. While use of the ABBI tool is believed to be a relatively effective way to perform a core biopsies of breast tissue, it is not apparently designed to remove cylinders of tissue having a diameter much in excess of about 20 mm. As such, it is not adapted for use in surgeries involving the removal of relatively large tissue portions in a single cutting sequence. In addition, the effectiveness of the ABBI tool in therapeutic, rather than diagnostic, surgeries has not been confirmed.

Detectors are used to locate organs or other portions of the body that have taken up a radioactive material, *e.g.*, an antibody labeled with a radioactive material, For example, the gamma ray probe described in U.S. Patents Nos. 5,170,055 and 5,246,005, both to Carroll et al., and sold by Care Wise Medical Products Corporation, Morgan Hill, California, and identified by the, trademark C-TRAK, provides an audio output signal, the pitch of which varies with changes in relative proximity between the probe and a body portion that has taken up an antibody labeled with a gamma ray producing material, *e.g.*, technetium 99. Once the body portion is detected, it is removed by known surgical techniques.

WO-A-98 38908 discloses a method and apparatus for obtaining and displaying an image of an object obtained by one modality such that the image corresponds to a line of view established by another modality.

WO-A-97 12553 discloses an apparatus for marking a predetermined margin around a tumour mass.

Even with the systems and techniques described above, it remains difficult for a surgeon to remove a tissue mass in amorphous, pliable tissue, such as breast tissue, so as to ensure that the entire tissue mass is removed while at the same time conserving portions of adjacent tissue. As a rewet, more tissue surrounding the targeted tissue mass is typically removed than is desired.

SUMMARY

The present invention is directed to a tissue anchor according to Claim 1 and a tissue stabilizer system according to Claim 9.

BRIEF DESCRIPTION OF THE DRAWINGS

Figure 1 is an isometric view representative of a tissue mass and surrounding tissue volume that is bracketed by the markers, with two markers being positioned on opposite ends of each of mutually orthogonal X, Y and Z-axes intersecting the tissue mass so as to define the boundary of the tissue volume, and with the probe and detector being positioned adjacent the tissue volume in accordance with one embodiment of the invention.

Figure 1A is an isometric view of the tissue mass illustrated in Figure 1, with two markers being positioned on opposite ends of each of mutually orthogonal X1, Y1 and Z-axes and with two markers being positioned on opposite ends of mutually orthogonal X2 and Y2-axes which are mutually orthogonal with respect to the Z-axis and offset along with Z-axis with respect to the X1 and Y1-axes.

Figure 1B is an isometric view of the tissue volume illustrated in Figure 1, with two markers being positioned on opposite ends of each of V, W, X and Y-axes, all of which lie in a common plane and are mutually orthogonal with respect to a Z-axis, all of these axes intersecting the tissue mass.

Figures 2a-2g are schematic representations of various examples of markers and their associated detection characteristics

Fig 2c is a representation of a marker used in the tissue anchor according to the invention.

Figure 3a is a block diagram of the elements of one embodiment of the marker illustrated in Figure 2c.

Figure 3b is a block diagram of the RF exciter used with the marker illustrated in Figure 3a.

Figure 4 is a block diagram of the elements of one example of the marker illustrated in Figure 2e.

Figure 5 is a block diagram of the RF exciter used with the marker illustrated in Figure 4.

Figure 6 is a perspective view of one example of the marker illustrated in Figure 2F, with details of internal construction being illustrated in phantom view.

Figure 7 is a block diagram of the probe and detector used with the marker illustrated in Figure 2b.

Figure 8 is a block diagram of the probe and detector used with the marker illustrated in Figure 2c.

Figure 9 is a front elevation view of a tissue anchor in accordance with one embodiment of the invention, with the cannula and rod of the cutter being shown in broken view to facilitate illustration.

Figure 10 is an enlarged view of the tissue anchor in Figure 9, with the rod and cannula both being broken at first location and the rod alone being broken at a second location to facilitate illustration, also with the rod being shown in a retracted position relative to the cannula.

Figure 11 is similar to Figure 10, except that the rod is shown in the extended position relative to the cannula, with the anchor members attached to the end of the rod being shown in an extended position engaged in a portion of a tissue mass.

Figure 12 is a top view of a breast of woman in a supine position, with a tissue mass being surrounded by markers of one embodiment of the present invention so as to define the tissue volume to be removed, and with an incision formed in the skin of the breast above the tissue volume.

Figure 13 is a cross-sectional view of the breast of Figure 12 taken along line 13-13 in Figure 12.

Figure 14 is similar to Figure 12, except that the skin adjacent the incision has been pulled apart to provide access to underlying breast tissue.

Figure 15 is an enlarged view of the incision of Figure 14, with the tissue anchor illustrated in Figures 9-11 being positioned in the tissue mass, and the two portions of a cutter illustrated being positioned adjacent the surgical cavity.

Figure 16 is similar to Figure 13, except that an incision has been formed in the skin of the breast and retracted to provide access to the underlying tissue mass to be removed and the tissue anchor has been positioned above the breast.

Figure 17 is an enlarged view of the portion of the breast illustrated in Figure 16 containing the tissue mass to be removed, with the tissue anchor being positioned in the tissue mass in the extended position so that the anchor members of the tissue anchor engage the tissue mass.

Figure 18 is similar to Figure 15, except that two portions of a cutter are illustrated in engaged, cooperative relationship and are positioned under the skin in contact with the tissue volume to be removed.

Figure 19 is similar to Figure 16, except that the tissue cutter is illustrated surrounding the tissue anchor and in cutting engagement with the tissue volume to be removed.

Figure 20 is similar to Figure 19, except that the tissue volume has been completely removed from the breast and is illustrated immediately above the surgical opening in engagement with the tissue anchor and cutter.

Figure 21 is an isometric view of a system for locating and defining a target location within a human body.

Figure 22 is a schematic elevation view illustrating a portion of a system for locating and defining a target location within a human body.

Figures 23A-D are isometric cut-away views of wireless resonating markers.

Figures 24-30 are side elevation views of several wireless implantable markers.

Figures 31-33 are side elevation views of several wireless implantable markers.

Figures 34-39 are isometric views of arrangements for implanting the wireless implantable markers relative to a target location T in accordance with embodiments of the invention.

Figures 40-43 are side cut-away views of instruments.

Figures 44 and 45 are schematic views of wireless controls for instruments.

Figures 46-51 are isometric views illustrating several instruments.

Figure 52 is an isometric view of a tissue anchor in accordance with another embodiment of the invention, illustrating the tissue fastener in an anchoring position.

Figure 53 is a side view of the tissue anchor of Figure 52 with the tissue fastener in a deployment position.

Figures 55-58 are isometric views similar to Figure 52, but showing tissue anchors in accordance with several alternative embodiments of the invention.

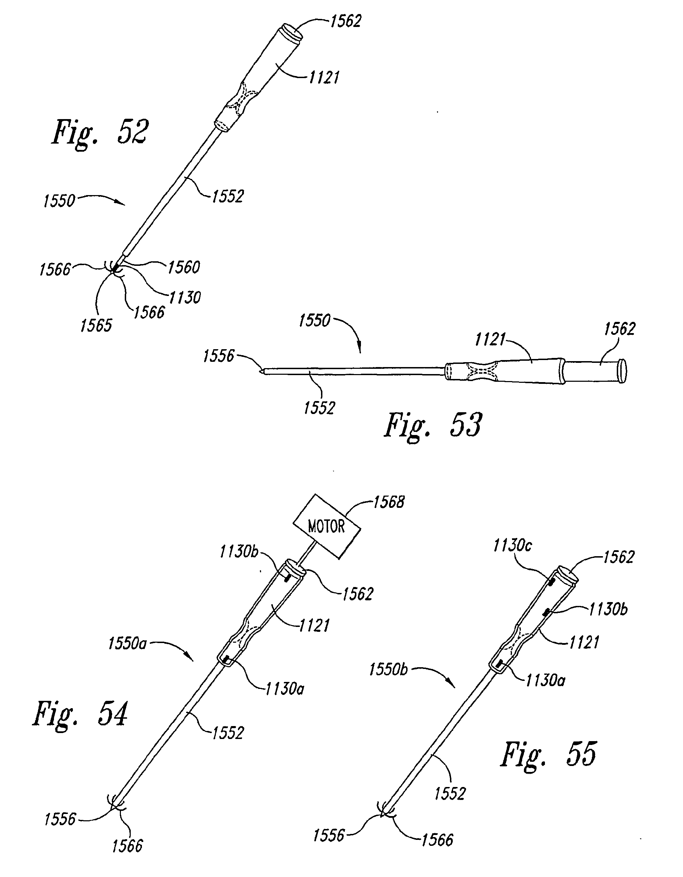

Fig. 54 shows a tissue anchor not forming part of the present invention

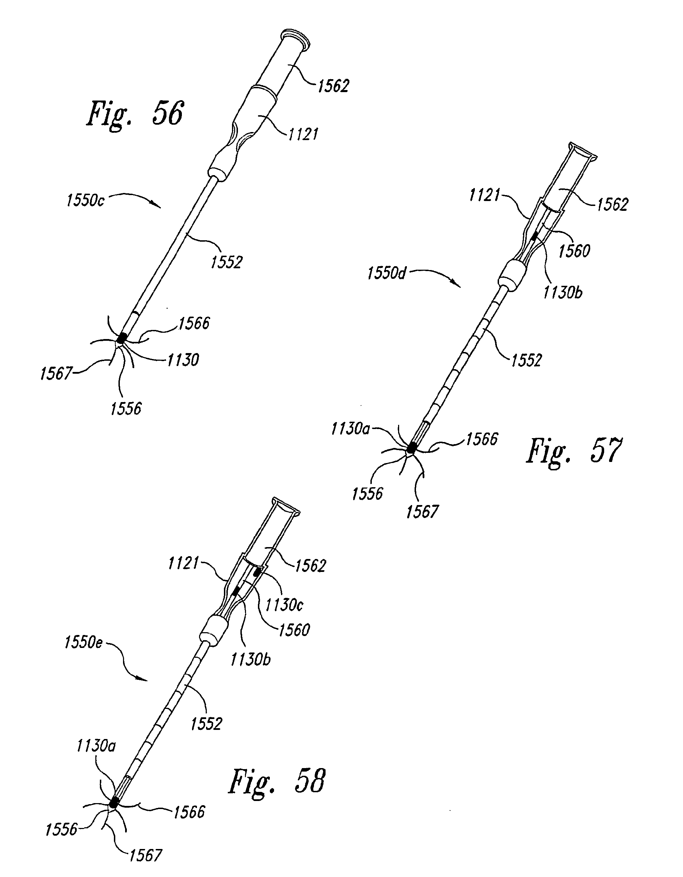

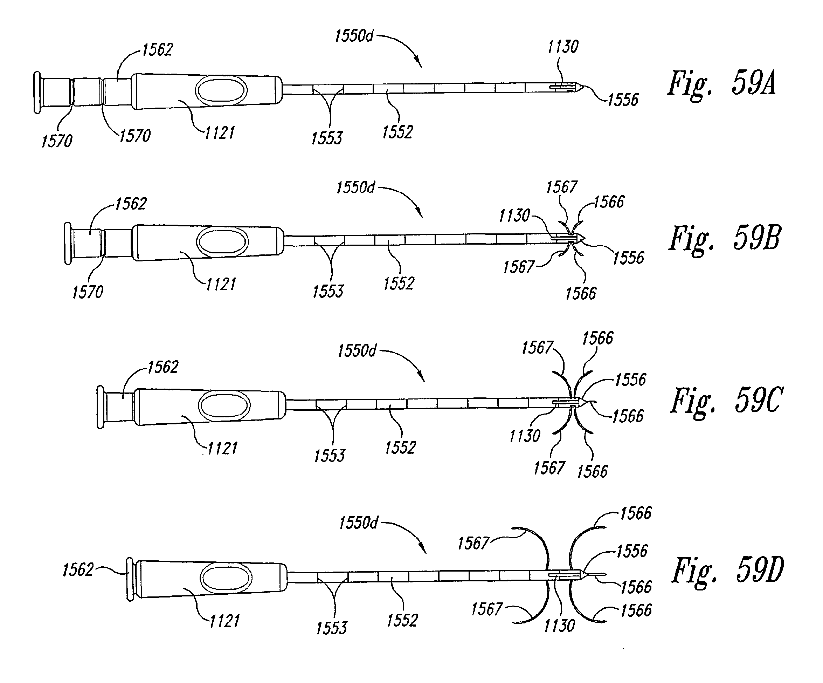

Figures 59A-59D illustrate the tissue anchor of Figure 56 with the anchor members in several different positions.

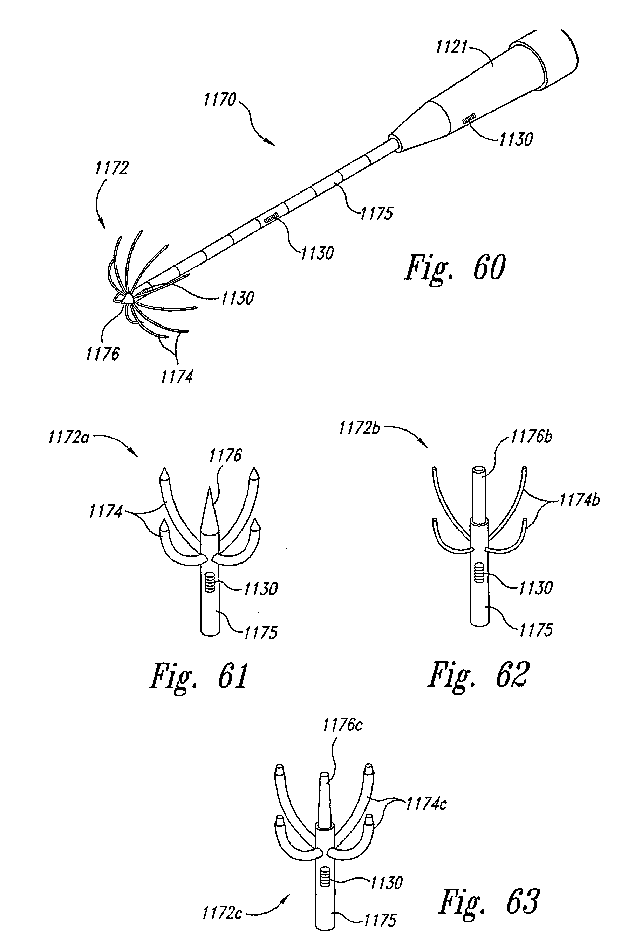

Figure 60 is an isometric view of an RF ablation device.

Figures 61-63 are isometric views illustrating several RF ablators.

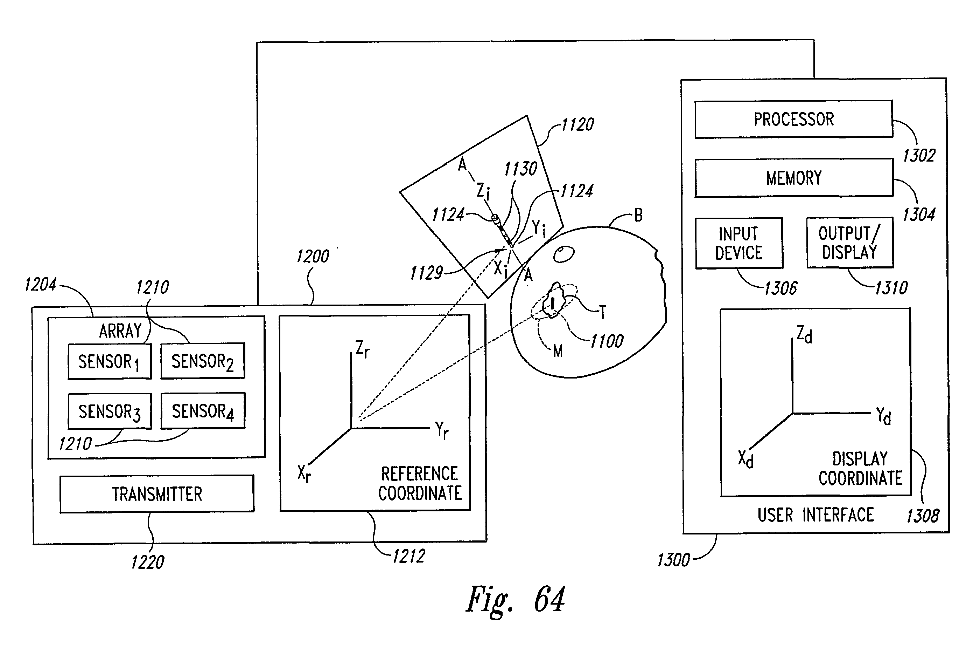

Figure 64 is a partially schematic view illustrating an aspect of operating a system for locating and defining a target location within a human body

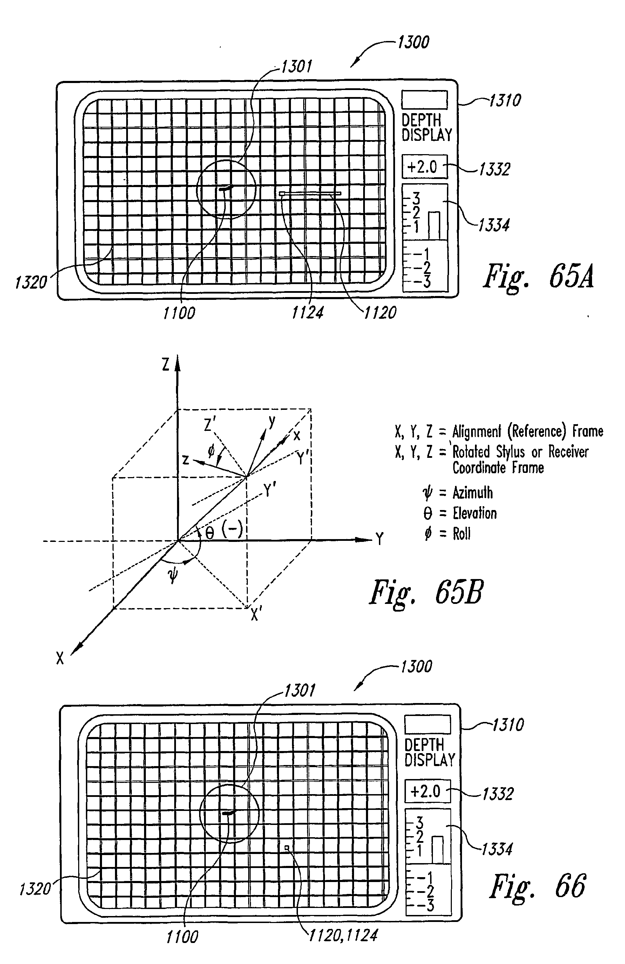

Figure 65 A is a front elevation view of an embodiment of a user interface.

Figure 65B is a graphical representation of a calibrating display coordinate system.

Figures 66-68 are front elevation views of several examples of user interfaces.

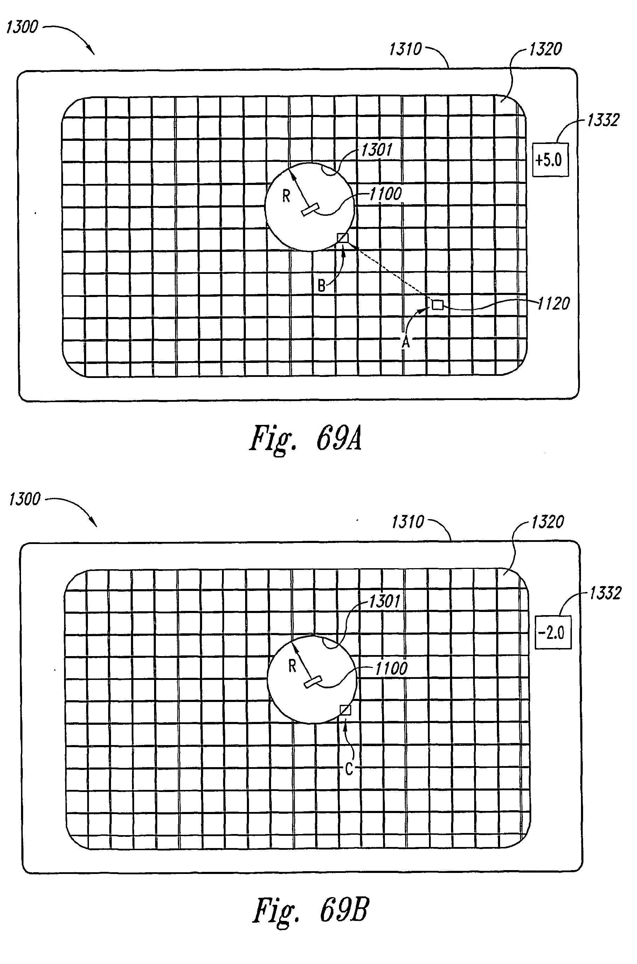

Figures 69A-69C are front elevation views of an example of a user interface illustrating a method of operating the system.

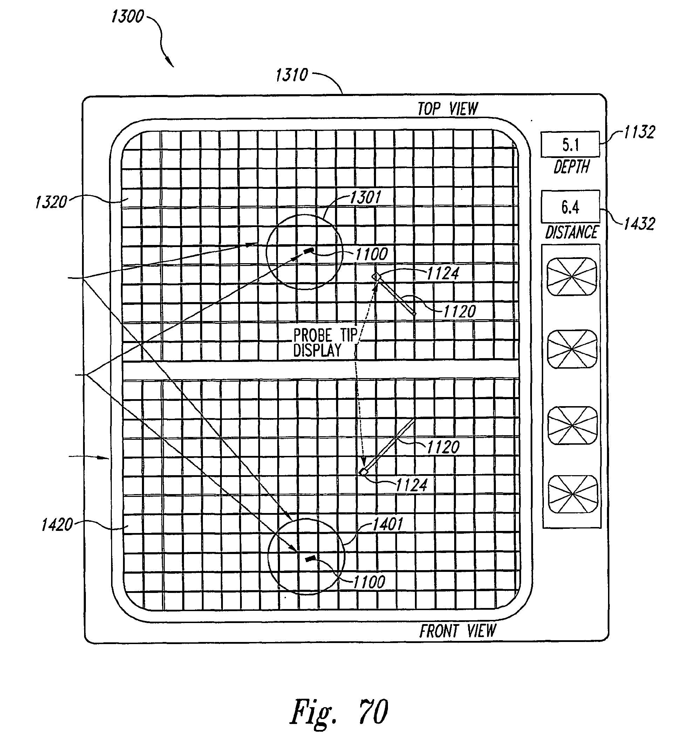

Figures 70-72 are front elevation views of several additional user interfaces

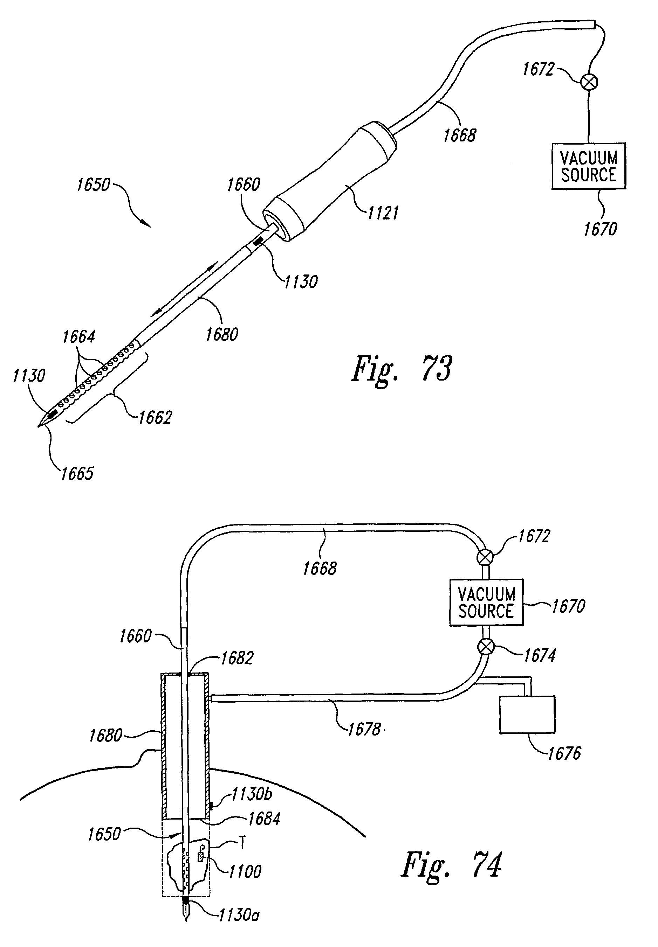

Figure 73 is an isometric view of a tissue anchor in accordance with another embodiment of the invention.

Figure 74 is a schematic illustration showing in partial cross section an instrument used in conjunction with the tissue anchor of Figure 73.

DETAILED DESCRIPTION

The following description is directed toward systems for locating and defining a target location within a human body. Several aspects of one system directed toward bracketing a target location with at least one marker are described below in Section I. Similarly, aspects of other systems directed toward locating a target mass within a human body using the relative orientation between an implanted marker and an instrument are described below in Section II. Other aspects directed toward defining and displaying a virtual boundary relative to a target location based on the location of an implanted marker are also described below in Section II.

I. Systems and Methods for Delineating a Target Location Using Bracketing

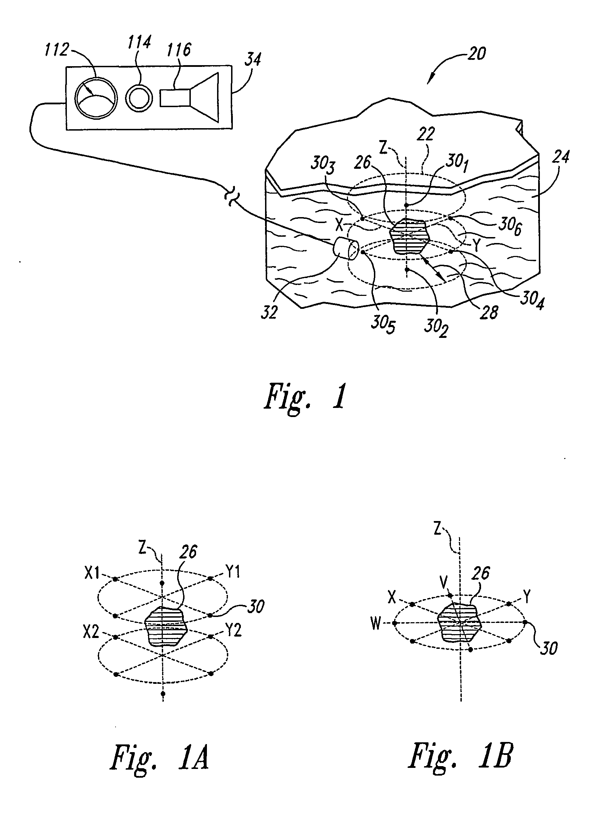

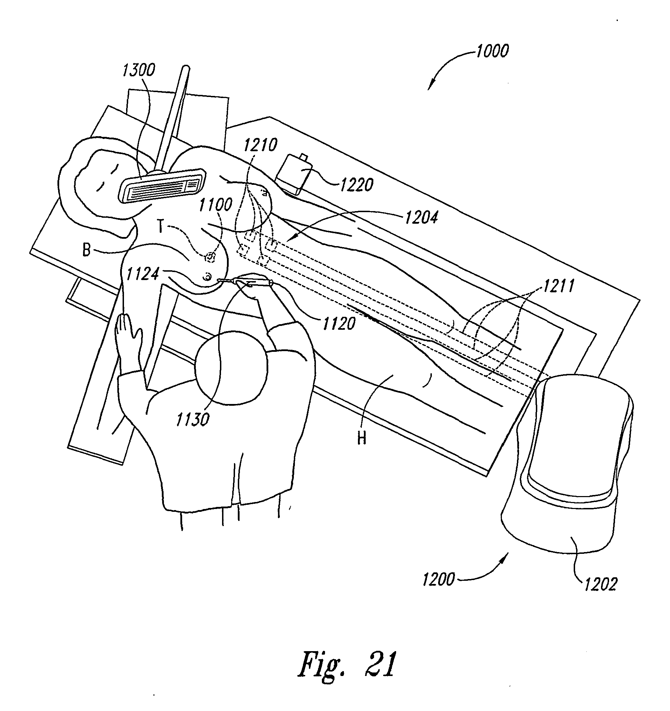

Figures 1-20 illustrate a system and several components for delineating a target location within a human body. Several of the components described below with reference to Figures 1-20 can also be used in the systems set forth with respect to Figures 21-61. Therefore, like reference numbers refer to like components and features throughout the various figures.

Referring to Figure 1, disclosed is a system 20 for defining the boundaries of, *i.e.*, bracketing, a tissue volume 22 in a tissue portion 24. Typically, tissue volume 22 will include a tissue mass 26, *e.g.*, a breast lesion, that is targeted for removal and a tissue margin 28 of unaffected tissue surrounding the tissue mass. After tissue volume 22 is bracketed, system 20 can be used to locate the defined boundaries of the tissue volume. *e.g.*, in connection with the surgical removal of tissue mass 26. It will be appreciated that the system can have other applications including radiation therapy, colo-rectal treatments, and many other applications in which it is useful to locate a target location other than a tissue volume within a human body.

As described in more detail below, other aspects of the present invention are also directed to a system for bracketing tissue volume 22 using system 20, and removing tissue volume 22 using system 20. These systems can be accomplished with the aspects of the present invention, such as stabilizers/anchors.

System 20 comprises a plurality of markers 30, a probe 32 and a detector 34 connected to the probe. As described in more detail below, markers 30 are implanted in tissue portion 24 under the guidance of a conventional imaging system not forming part of the present invention, so as to bracket tissue volume 22. Such imaging systems may include ultrasound, magnetic resonance imaging ("MRI"), computer-aided tomography ("CAT") scan, and X-ray systems. Markers 30 are imageable with the imaging energy generated by the imaging system. For example, if an ultrasound imaging system is used to implant markers 30, the latter are configured and made from a material that strongly reflects ultrasound energy. Materials that are imageable with the energy generated by such systems are well known to those skilled in the art, and so are not described in detail here. Following implantation of markers 30, probe 32 and detector 34 are used to locate the markers, as described in more detail below.

The terms "probe 32" and "detector 34" are used generically herein to refer to all examples of the probe and detector described below. Specific examples of the probe 32 and detector 34 are identified using a prime notation described below, *i.e.*, probe 32' or detector 34". Additionally, the probes described below define one type of instrument, and the detectors described below define one type of position detection system.

A. Markers

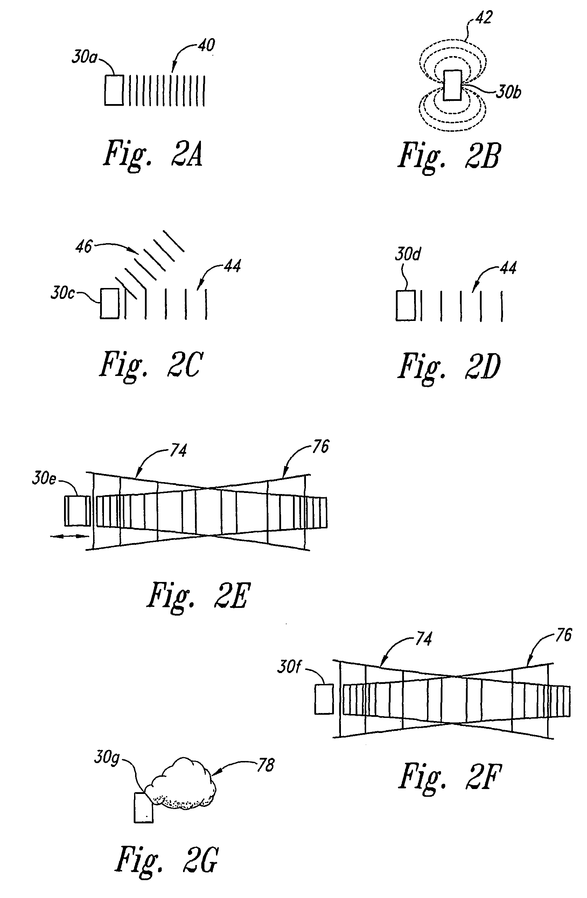

The markers 30 can be biologically inert (biocompatible) and are relatively small so that they do not impair procedures for removing or treating a tissue volume 22. Markers 30 may have different geometric configurations, *e.g.*, spherical, disk-like, cylindrical, and other shapes. In one particular example, the greatest dimension of a marker 30 measured along a Y-axis extending through the marker from one surface to an opposite surface is not more than about 5 mm. The markers 30 can be even smaller, *e.g.*, the greatest dimension is about 1-2 mm, or they can also be larger. Although several of the markers with respect to Figures 2-8 are described in connection with this aspect of the invention, they can also be used in connection with other aspects.

In addition, markers 30 each have a detection characteristic to enable detection by probe 32 and detector 34, or by a separate detection system with an array of sensors relative to a reference location. The detection characteristics of the various embodiments of markers 30 can be characterized as active or passive. In the active category, the detection characteristic of a first marker 30, illustrated in Figure 2a as marker 30a, is gamma radiation 40. In this regard, marker 30a may include materials such as technetium 99, cobalt isotopes or iodine isotopes. Such materials may be obtained from DuPont of Billerica, Massachusetts. Preferably, each marker 30a generates gamma radiation 40 having a field strength in the range of 1-100 microCuries.

Also in the active category, in a second example of marker 30, illustrated in Figure 2b as marker 30b, the detection characteristic is magnetic field 42. Markers 30b of the second example thus contain ferromagnetic materials in which a magnetic field can be induced, or alternatively are permanently magnetized and so have an associated permanent magnetic field. In Figure 2b, magnetic field 42 represents both the induced and inherent magnetic fields. Strong permanent magnets, such as those made from Samarium-Cobalt, can be suitable magnets for markers 30b.

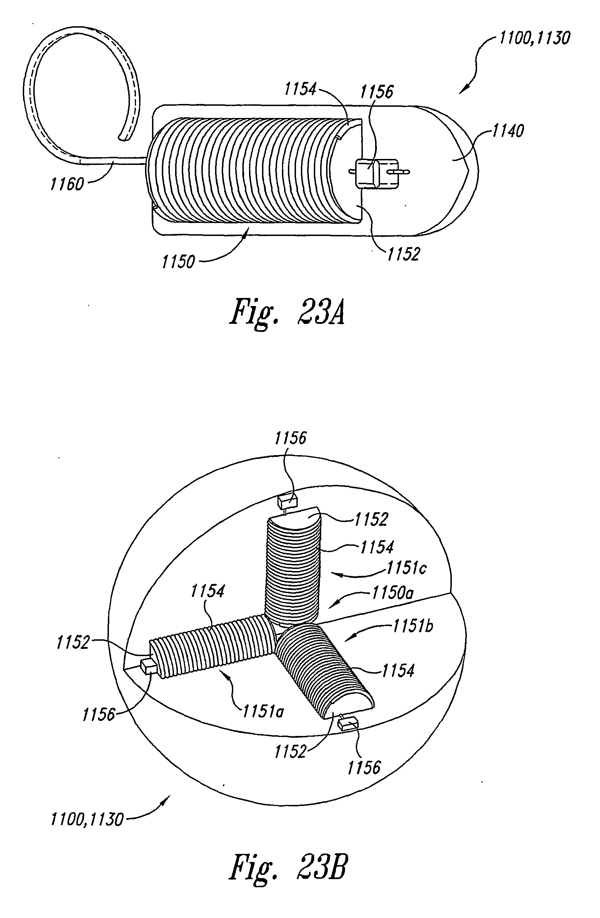

Alternatively, the markers may communicate with the position detection system by resonating markers (*e.g.*, AC magnetic coupling using coils of wire as receiving and emitting antenna) as described with reference to Figures 23A-D.

Referring to Figure 2c a marker (30c), used in the tissue anchor according to the present invention, again in the active category, emits radio frequency ("RF") signal 44 in response to a triggering signal 46. Various energy sources may be used for triggering signal 46, including a magnetic field, ultrasound or radio frequency energy. In this latter case, marker 30c is designed to receive triggering signal 46 which has a first RF wavelength, and in response thereto, emit signal 44 of a second RF wavelength. In the simplest case, no data, other than the specific radio frequency itself, is carried in signal 44. Alternatively, markers 30c may all transmit signal 44 at a single frequency, with data uniquely identifying each marker being carried in signal 44 emitted by each marker.

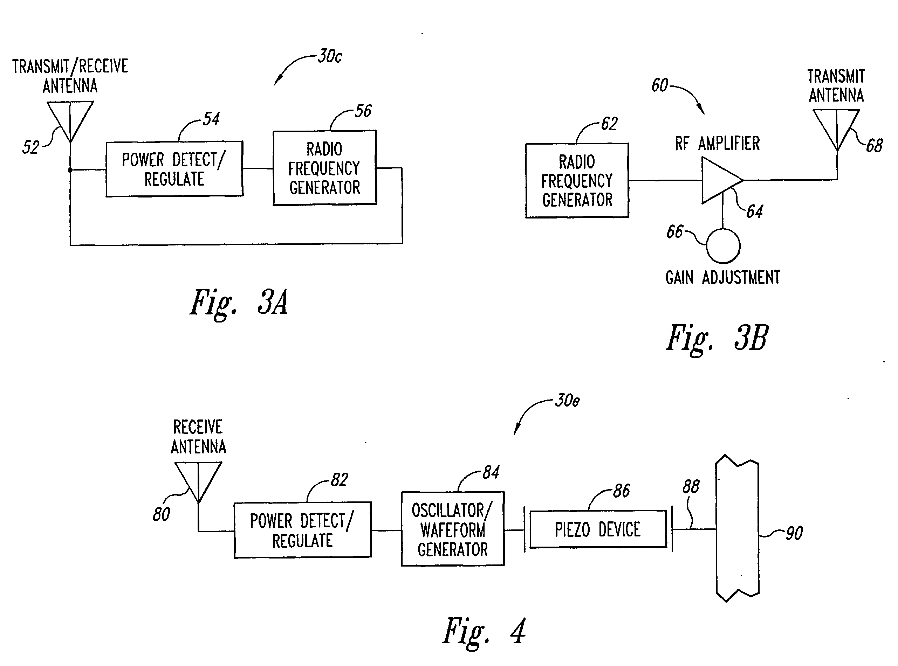

A suitable marker 30c is illustrated in Figure 3a. This marker 30c includes a transmit/receive antenna 52 for receiving an RF signal at a first frequency and transmitting an RF signal at a second frequency. Also included is a power detect/regulate circuit 54 connected to antenna 52 that detects the presence of, and regulates, the RF signal received by the antenna. The regulated RF signal is provided from circuit 54 to drive radio frequency generator 56 which generates an RF signal at a second frequency. As discussed in more detail below, when multiple markers 30c are used together in a given bracketing procedure, preferably each marker transmits RF signals at a second frequency which is unique to the marker. The frequency of the received RF signal 46, however, is preferably common with respect to all of the markers 30c used in the bracketing procedure. The RF signal generated by radio frequency generator 56 is then provided to antenna 52 where it is transmitted as an RF signal.

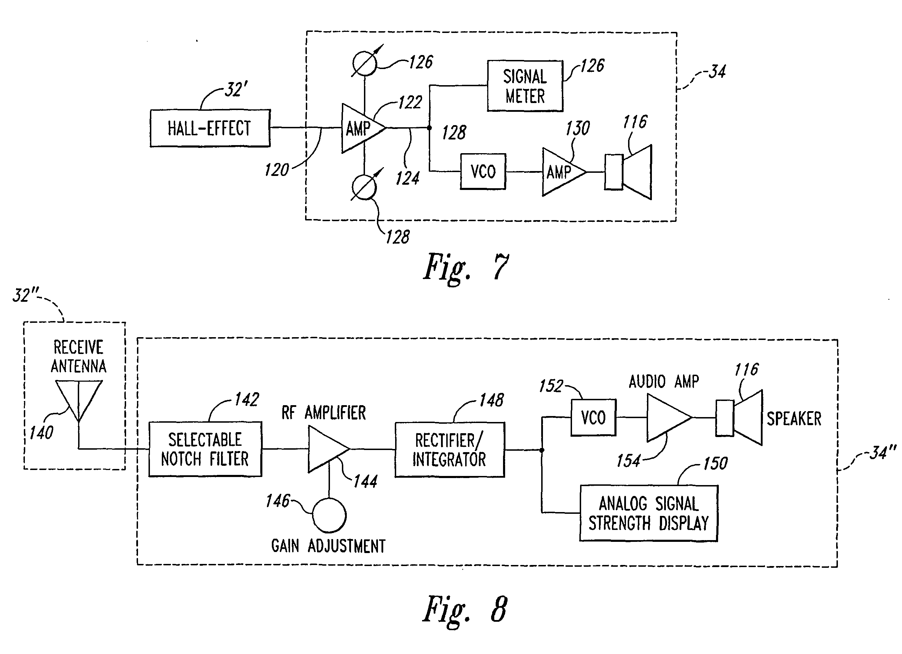

Referring to Figure 3b, an RF exciter device 60 for generating RF signal 46 is illustrated. RF exciter 60 includes a radio frequency generator 62 for generating RF signal 46 at a predetermined frequency and an RF amplifier 64 for amplifying the output from the radio frequency generator. The sensitivity of amplifier 64 may be controlled using gain adjustment 62 coupled to the amplifier. The output of RF amplifier 64 is provided to transmit antenna 68 which transmits RF signal 46. Transmit antenna 68 of RF exciter 60 is preferably placed in relatively close proximity to marker 30c, with appropriate gain adjustment of RF amplifier 64 being achieved by control gain adjustment 66 until a suitable return signal is absorbed from detector 34", discussed below and illustrated in Figure 8.

In a fourth example, not used in the tissue anchor according to the present invention, again in the active category, marker 30d, illustrated in Figure 2d, continuously emits signal 44 at specific frequencies in the radio frequency spectrum. The marker 30c illustrated in Figure 3A and described above can be satisfactorily employed as marker 30d by adding a battery (not shown) in place of power detector portion of circuit 54 of marker 30c. RF exciter 60 is not required in connection with marker 30d, insofar as the battery generates the energy used by the marker in producing RF signal 44. The examples of the RF Markers are one example of a resonating marker having an electrical circuit in accordance with an embodiment of a wireless implantable marker.

As a fifth example in the active category, marker 30e, illustrated in Figure 2e, is designed to vibrate following implantation. This vibration is a detection characteristic that is chosen to enhance image contrast when marker 30 is intended to be detected using a probe 32 and detector 34 that perform ultrasound imaging. More specifically, incoming ultrasound signal 74 is reflected off marker 30e as reflected ultrasound signal 76, with a Doppler shift component being added to the reflected signal due to the vibration of the marker to enhance imageability of the marker. The vibration frequency of marker 30e will vary depending upon the frequency of ultrasound energy generated by probe 32, but is preferably lower than the frequency of incoming ultrasound signal 74 which is typically 7.5 MHz, *i.e.*, the vibration frequency is preferably in the 50 Hz to 50 kHz range. This example is an example of a mechanical resonating marker in accordance with another example of a-wireless implantable marker.

A suitable marker 30e that achieves the functionality described above is illustrated in Figure 4. This marker 30e includes an antenna 80 for receiving an RF signal that provides the energy driving the marker. A power detection and regulation circuit 82 is connected to antenna 80 for detecting when the antenna is receiving an RF signal and for regulating the signal for use by oscillator and waveform generator circuit 84 connected to circuit 82. Circuit 84 converts the regulated RF signal received from circuit 82 into an oscillating electrical signal, preferably in the audio frequency range (*i.e.*, 20 Hz-20 kHz), having a waveform that is optimized to drive piezoelectric device 86 connected to circuit 84. Piezoelectric device 86 is a conventional piezoelectric device of the type that converts an oscillating electrical input signal into mechanical oscillations. Piezoelectric device 86 is attached via support 88 to outer housing 90 of marker 30e. Housing 90 is designed to resonate at the mechanical oscillation frequency of piezoelectric device 86.

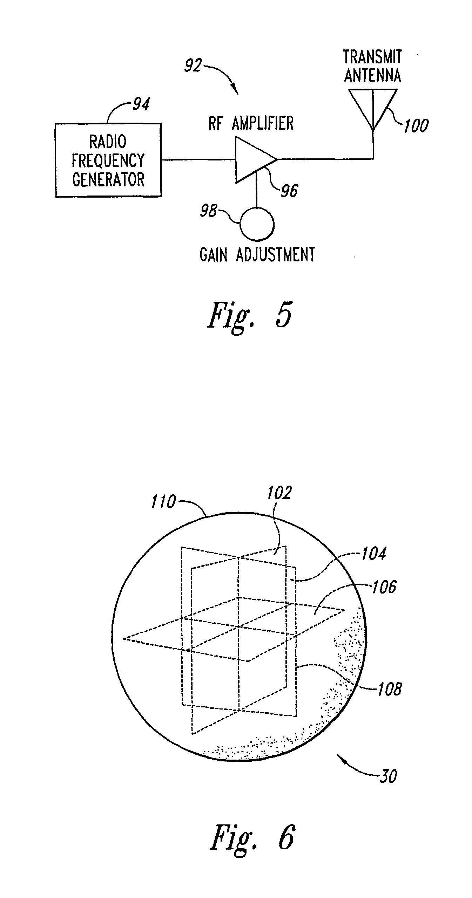

Referring to Figure 5, an RF coupled acoustic exciter 92 is provided for generating the RF signal received by antenna 80 of marker 30e. Exciter 92 includes a radio frequency generator 94 for generating an RF signal. RF amp 96, with a gain adjustment 98 connected hereto, is provided for receiving and amplifying the output signal from generator 94. A transmit antenna 100 is provided for receiving the output of amp 96 and transmitting the RF signal used to drive marker 30e. In use, gain 98 of amp 96 is adjusted to amplify the RF signal produced by generator 94 such that marker 30e is caused to mechanically oscillate so it is most clearly observable by the ultrasound imaging system (not shown) used in conjunction with marker 30e.

As those skilled in the art will appreciate, other circuit configurations may be used in marker 30e to cause piezoelectric device 86 to vibrate. For example, a frequency divider circuit (not shown) may be used in place of oscillator/ waveform generator circuit 84. With such alternative, exciter 92 is modified to include a variable frequency oscillator (not shown) in place of radio frequency generator 94..

In the passive category (details of which are provided for comparison purposes only), the detection characteristic in a sixth example of marker 30, illustrated as marker 30f in Figure 2f, is opacity to incoming ultrasound signal 74. That is, marker 30f reflects incoming sound energy sufficiently to create a strong image in reflected signal 76 so as to enhance imageability using a conventional ultrasound imaging system. In many cases, it will be advantageous to incorporate the detection characteristics of marker 30f in marker 30e.

While those skilled in the art are familiar with materials and configurations that can be used for marker 30f, one suitable marker 30f is illustrated in Figure 6. This marker 30f includes plate 102, plate 104 and plate 106, all of which are preferably arranged in mutually orthogonal relationship. It is preferred that each of the plates 102-106 has a square configuration and the length of each edge of the plates, *e.g.*, the length of edge 108 of plate 104, is preferably about twice the wavelength of incoming ultrasound signal 74. For example, when incoming ultrasound signal 74 has a wavelength of 7.5 MHz, edge 108 has a length of about 2 mm. Plates 102-106 are made from a material that strongly reflects ultrasound energy, *e.g.*, aluminum, and typically have a thickness in the range of 10-100$\mu$m. Plates 102-106 ideally are enclosed in a biologically non-reactive casing 110. The latter is preferably made from a material that does not have strong ultrasound reflection characteristics, e.g., a soft polymer.

Also in the passive category (details of which are provided for comparison purposes only), marker 30g of the seventh example, illustrated in Figure 2g, comprises a capsule (not shown) filled with a colored dye 78, *e.g.*, a vital dye. Either or both the capsule and dye 78 of marker 30g are made from a material that is imageable by the imaging system, *e.g.*, ultrasound, used to implant the markers, as described in more detail below. The capsule is made from gelatin or other suitable material that is selected to be sufficiently tough to withstand insertion into tissue volume 22, but is relatively easily cut by the cutting tool used to remove the tissue volume, *e.g.*, a conventional surgical scalpel or cutting tool 200 described below. Marker 30g provides a visual guide as to its location by releasing colored dye 78 when severed by a surgical cutting tool, In,this regard, probe 32 and detector 34 are not used in connection with marker 30g.

Markers 30a, 30b and 30f may be made from a solid structure containing material having the desired detection characteristic. Alternatively, markers 30a, 30b and 30f may be made from a capsule filled with a dye, such as is used for marker 30g. containing material having the desired detection characteristic. As another alternative, all of markers 30 may include a dye contained in an outer capsule having the requisite toughness and severability characteristics noted above.

B. Probe and Detector

The probe 32 shown in Figure 1 is one example of an instrument, and the detector 34 shown in Figure 1 is one embodiment of a user interface for any system in accordance with the invention. The design and configuration of the probe 32 and the detector 34 depend upon the marker 30 used. However, for all of marker 30 (except marker 30g), detector 34 is designed to provide humanly recognizable information when probe 32 is positioned within a selected proximity, *e.g.*, 1-5 cm, of a given marker. This information may take one of a variety of forms, including a burst of humanly perceivable sound, constant or intermittent illumination of a light, movement of a needle on a dial, a short burst of air, change of data in a visual display, increased image brightness or contrast (in the case when detector 34 is an ultrasound imaging system, as discussed below), a tactile response, or other humanly perceivable proximity information. In this regard detector 34 may include a dial 112, light 114, speaker 116, or other appropriate devices for generating the selected form of humanly perceivable information.

Preferably, although not necessarily, detector 34 provides humanly recognizable information that indicates changes in proximity of probe 32 to a given marker 30. Thus, rather than merely providing static or threshold information that probe 32 is within a predetermined range of a given marker 30, detector 34 preferably provides proximity information having an attribute or characteristic that varies as a function of changes in proximity of the probe relative to the marker. For example, if the proximity information is sound, the pitch is varied with changes in proximity. Or, as another example, if the proximity information is light, the brightness of the light changes with changes in proximity.

A probe and detector that may be satisfactorily employed as probe 32 and detector 34, respectively, when the latter is intended to detect maker 30a, is sold by Care Wise Medical Products Corporation of Morgan Hill, California, and is identified by the trademark C-TRAK. The C-TRAK probe, which is described in U.S. Patents Nos. 5,170,055 and 5,246,005 to Carroll et al., provides a humanly audible sound, the pitch of which varies with changes in proximity of the probe to tissue labeled with gamma ray producing material.

Referring to Figures 1, 2b and 7, when probe 32 and detector 34 are intended for use in detecting marker 30b, which generates a magnetic field 42, probe 32' and detector 34' illustrated in Figure 7 may be satisfactorily employed. Probe 32' includes a conventional Hall effect sensor (not shown) that provides an output signal on line 120, the voltage of which varies as a function of proximity of the probe to the magnetic field generated by a marker 30b. Detector 34' is connected to probe 32' via line 120, and includes an amplifier 122 connected to line 120 for amplifying the signal from the Hall effect sensor in probe 32'. Amplifier 122 includes an offset adjustment 126 and a gain adjustment 128. Offset adjustment 126 is provided to cancel the effects of any ambient magnetic fields, such as that of the earth. Gain adjustment 128 is provided to control the overall sensitivity of detector 34'. The amplified signal from amplifier 122 is delivered on line 124 to signal meter 126, which may comprise a dial with a movable needle, an LED or other device for representing signal strength. Also connected to line 124 is voltage-controlled oscillator 128, the output of which is provided to amplifier 130. The output of amplifier 130 drives speaker 116. The frequency of the output signal from voltage controlled oscillator 128 varies as function of changes in voltage of the signal delivered on line 124, which in turn causes the pitch of the sound produced by speaker 116 to vary as a function of changes in the voltage of the signal on line 124. As those of ordinary skill in the art will appreciate, other devices for providing humanly recognizable information representing changing proximity, *e.g.*, a light, may be employed instead of speaker 116.

Referring to Figures 1, 2c and 8, for markers 30c and 30d, which generate radio frequency energy, probe 32" and detector 34" are provided for use in detecting the markers. Probe 32" includes a conventional coil antenna 140 for receiving an RF signal. Detector 34" includes a selectable notch filter 142 connected to antenna 140 which permits tuning of the detector to the unique RF frequency of signal 44 emitted by markers 30c or 30d. A tuning knob or other user adjustable mechanism (neither shown) is attached to selectable notch filter 142 to permit a user to perform such tuning. The output of selectable notch filter 142 is provided to RF amplifier 144, the overall sensitivity of which may be controlled by gain adjustment 146 attached to the amplifier. The output of RF amplifier 144 is provided to rectifier/ integrator circuit 148 which rectifies and time filters the signal. The output of rectifier/integrator circuit 148 is provided to analog signal strength display 150 which provides a visual indication of the proximity of probe 32" to marker 30c. In addition, the output of rectifier/integrator circuit 148 is provided to voltage oscillator 152 which generates an output signal, the frequency of which varies as a function of the voltage level of the signal provided by rectifier/integrator circuit 148. The output signal of the voltage control oscillator 152 is amplified by audio amplifier 154, which in turn drives speaker 116. Accordingly, the pitch of the sound generated by speaker 116 varies as a function of the strength of the RF signal received by probe 32", and hence as a function of the proximity of probe 32" to markers 30c or 30d.

A suitable probe 32 and detector 34 for use with the markers 30e and 30f is the ultrasound imaging system available from Dornier Surgical Products, Inc., Phoenix, Arizona, is identified by the name Performa, and generates ultrasound energy having a frequency of 7.5 MHz.

C. Tissue Anchor

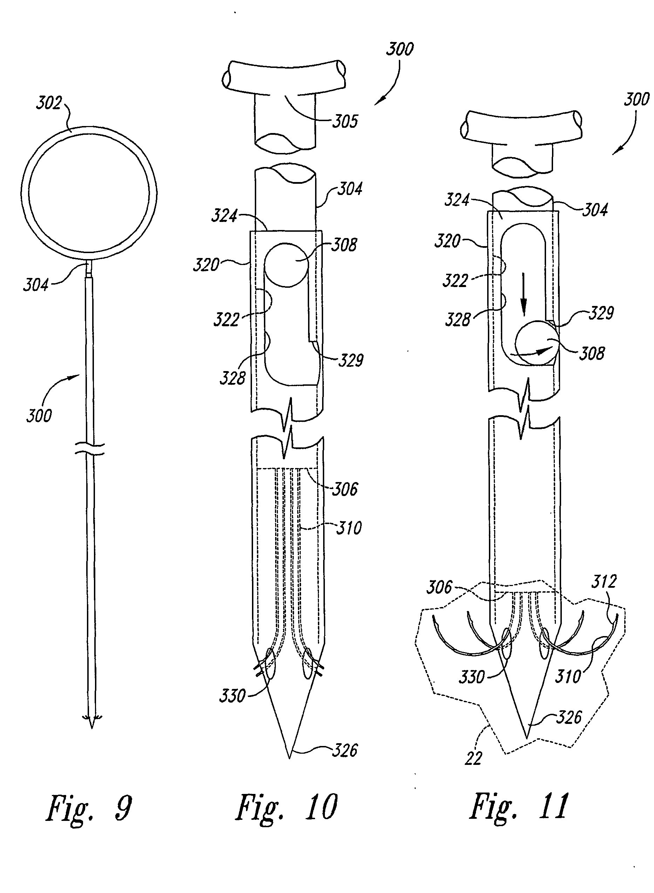

Turning now to Figures 9-11, a tissue anchor 300 of the present invention is described. The latter is designed to stabilize tissue mass 26 during surgical removal of the mass using system 20, as described in more detail below.

Tissue anchor 300 includes a ring 302 sized to receive the thumb or finger of a user, and a lead or rod 304. The latter includes a proximal end 305, which is attached to ring 302, and a distal end 306. Rod 304 includes an outwardly projecting pin 308 that serves as a stop, as described below. Tissue anchor 300 also includes a tissue fastener which is illustrated as a plurality of, *e.g.*, four, anchor members 310 that are attached to rod 304 at or adjacent its distal end 306. Typically, anchor members 310 are attached to rod 304 so as to extend away from its distal end 306, as illustrated in Figures 9 and 10. However, as an alternative design, anchor member 310 may be attached to rod 304 so as to extend away from distal end 306 toward proximal end 305 (not shown). Each anchor member 310 may terminate with a barb 312 (Figure 11), if desired. Anchor members 310 preferably have a curved configuration when in an unbiased state, as illustrated in Figures 9 and 11. Anchor members 310 are preferably made from spring steel, although other "memory" metal alloys made also be satisfactorily used. In certain applications it may be unnecessary to provide a curve in anchor member 310, *i.e.*, the anchor member may be substantially straight.

Rod 304 preferably, although not necessarily, has a circular cross section. The outside diameter of rod 304 depends upon its intended application, but is typically in the range of 0.3-10 mm, preferably about 1-2 mm. The length of rod 304, as measured between proximal end 305 and distal end 306, also depends upon its desired application, but typically ranges from 5-20 cm.

Tissue anchor 300 also includes an introducer, typified in Figures 9-11 as a cannula 320 having a central bore 322, a proximal end 324 and a pointed distal end 326. Central bore 322 has an inside diameter that is sized to receive rod 304 with a close sliding fit. Cannula 320 has an outside diameter that is selected based on the intended application but is typically in the range 0.5 mm-12 mm, preferably about 1-3 mm. Cannula 320 also includes an elongate slot 328 that runs parallel to the long axis of the cannula and is sized to receive pin 308 with a close sliding fit. The length of slot 328 is substantially the same as the length of anchor members 310. Slot 328 includes a pocket 329 at its end closest to distal end 326 of cannula 320 that extends orthogonally to the long axis of the slot and is sized to receive pin 308.

Cannula 320 also includes a plurality of apertures 330 extending through the wall of the cannula. Apertures 330 are positioned adjacent distal end 326 of cannula 320 when anchor members 310 are attached to rod 304 to extend away from distal end 306 as illustrated in Figures 10 and 11. If anchor members 310 extend from distal end 306 toward proximal end 305 (not shown), then apertures 330 are moved toward the proximal end so that they are spaced from the distal end 326 at least about the length of the anchor members. One aperture 330 is typically provided for each anchor member 310. The lengths of anchor members 310, cannula 320, and slot 328 are together selected so that a small portion, *e.g.*, about 1 mm, of each anchor member 310 projects from its respective aperture 330 when tissue anchor 300 is in the retracted position illustrated in Figure 10. In this position, pin 308 engages the end of slot 328 closest to proximal end 324. Anchor members 310 are sized in this manner to ensure the anchor members remain positioned in their respective apertures 330 when the anchor members are in the stored position illustrated in Figure 10.

The lengths of anchor members 310, cannula 320, and slot 328 are also together selected so that most, if not substantially the entire, length of the anchor members 310 projects from their respective apertures 330 when tissue fastener is in the anchoring position illustrated in Figures 9 and 11. In this position, pin 308 engages the end of slot 328 closest to distal end 326.

The elements of tissue anchor 300 are preferably made from stainless steel, a plastic such as polystyrene or polyurethane, or other materials suitable for the intended application of the tissue anchor (as described in more detail below) known to those skilled in the art. As noted above, in many cases it is desirable to make anchor members 310 from spring steel or a "memory" metal alloy.

D. Bracketing

Referring now to Figures 1, 12 and 13, markers 30 may be used to bracket (*i.e.*, define the boundaries of) tissue volume 22 in a tissue portion 24 in accordance with the following method. In the following description of the method of bracketing tissue volume 22, the latter is contained in a human breast. However, it is to be appreciated that tissue volume 22 may be present in other hollow or solid organs and structures, *e.g.*, a liver, or may constitute an entire organ or structure. Additionally, a plurality of the markers 30 may be implanted to completely bracket the tissue volume 22, or one or more markers 30 can be used to bracket or otherwise mark the location of the tissue volume 22.

As the first step in bracketing tissue volume 22, a tissue mass 26 of interest is identified through conventional imaging methods, *e.g.*, ultrasound, MRI, X-ray or CAT scan. Next, markers 30 are implanted in tissue portion 24 surrounding tissue mass 26 and defining outer boundaries of tissue volume 22. The number of markers 30 used, and the placement of the markers relative to tissue mass 26, will vary depending upon the location of the tissue mass relative to other types of tissue, *e.g.*, bone or muscle, surgeon preference, size and configuration of the tissue mass and the desired amount of tissue margin 28 (Figure 1) beyond the edge of tissue mass 26. However, in many applications, it may be desirable to use at least six markers 30 to bracket tissue volume 22, preferably two on each of axes X, Y and Z (see, *e.g.*, Figures 1, 12 and 13). Two of the markers 30 can be positioned on each of axes X, Y and Z so as to lie on opposite boundaries of tissue volume 22. For example, as illustrated in Figure 1, marker $30_1$ lies on the Z-axis at the upper surface of tissue volume 22, marker 302 lies on the Z-axis at the lower surface of the tissue volume, marker $30_3$ lies on the X-axis at a first location on the outer surface of the tissue volume, marker $30_4$ lies on the X-axis at a second location on the outer surface of the tissue volume diametrically opposite marker $30_3$, marker $30_5$ lies on the Y-axis at a third location on the outer surface of the tissue volume, and marker $30_6$ lies on the Y-axis at a fourth location on the outer surface of the tissue volume diametrically opposite marker $30_5$.

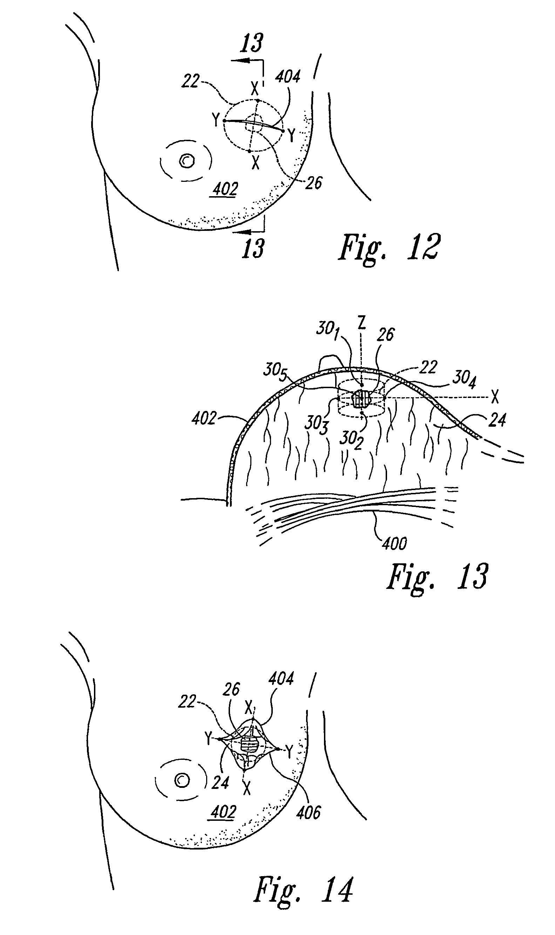

Although the axes X, Y and Z can be mutually orthogonal, as illustrated, this is not mandatory and can be difficult to precisely implement in practice. In this particular example, the tissue volume 22 should be completely surrounded by markers 30, *i.e.*, the tissue volume should be defined in three dimensions by the markers. One notable exception to this that the marker 30, such as marker $30_2$ shown in Figures 1 and 13, positioned at the base of, *i.e.*, underneath, tissue volume 22 is not typically required when a different type of tissue, such as pectoral muscle 400 (Figure 13) is located at or near where the marker would be positioned. The illustration of marker 302 in Figure 13 is not inconsistent with this recommended placement regime for markers 30 because of the relatively great spacing between the marker $30_2$ and pectoral muscle 400. Similarly, when the marker 30, such as marker $30_1$ shown in Figure 1, to be positioned on top of tissue volume 22 is near the skin overlying the tissue volume, such marker is not typically required. Also, while the X, Y and Z-axes are illustrated in Figure 1 as intersecting at a common point centrally located within tissue mass 26, this is not required. For example, it may be desirable to offset the X and Y-axes somewhat, as measured along the Z-axis. Furthermore, in some cases it may be desirable to define tissue volume 22 with markers 30 in only two dimensions or in only one dimension.

In some cases, it will be desirable to use more than two markers 30 on X, Y and Z-axes. Referring to Figure 1A, in a first case, ten markers 30 are used, two on the Z-axis, two on an axis $X_1$, two on an axis $X_2$ that is offset along the Z-axis with respect to axis $X_1$ two on an axis $Y_1$, and two on an axis $Y_2$ that is offset along the Z-axis with respect to axis $Y_1$. Referring to Figure 1B, in a second case, ten markers 30 are used, two on the X-axis, two on the Y-axis, two on the Z-axis, two on the V-axis which bisects the X and Y-axes and two on the W-axis which also bisects the X and Y-axes, but at a different location. Other numbers and relative placements of markers are also encompassed by the present disclosure.

Markers 30 are preferably spaced from tissue mass 26 so as to define tissue volume 22 such that tissue margin 28 is large enough to ensure none of the tissue mass of interest lies outside the tissue volume. This precise spacing will vary with the nature of the tissue mass 26, the size of the tissue mass, surgeon preference and other factors. However, tissue margin 28, as measured outwardly along an axis extending perpendicular to a surface location on tissue mass 26, is generally about 0.5 cm to 3 cm, and is preferably about 1 cm to 2 cm. It will be appreciated that other margins may be more appropriate in other circumstances.

Markers 30 may be implanted in tissue portion 24 in a variety of different ways using a variety of different tools. In general, markers 30 are implanted using a conventional imaging system (not shown) that simultaneously generates an image of tissue mass 26 and the markers. By frequently comparing the location of markers 30 to tissue mass 26 during implantation of the markers into tissue portion 24, based on image information received from the imaging system, the markers may be positioned so as to define tissue volume 22 in the manner described above. As noted above, markers 30 are made from a material that provides good image contrast with respect to the imaging energy used. In other aspects, only one or two markers may be implanted in or proximate to the tissue mass 26, and the margin 28 can be defined on a display by a virtual line or shape based upon the relative location between at least one of the implanted markers and the tissue mass 26.

It is preferable to at least partially immobilize tissue portion 24 during implantation of markers 30. However, this is not necessary because, by comparing the relative location of a marker 30 to tissue mass 26, the desired relative placement can typically be achieved even if tissue portion 24 is moving during marker implantation.

E. Marker Implantation

Various techniques may be used to implant markers 30 in tissue portion 24. With reference to Figures 12 and 13, one approach is to insert markers 30 percutaneously through skin 402 overlying tissue portion 24 using known needle pushers or implanters (neither shown) of the type used to implant "seeds" of radioactive material for various cancer treatments. For example, needle pushers of the type sold by Best Industries of Springfield, Virginia, may be satisfactorily employed. These needle pushers include a central needle surrounded by an outer tube having an end plate or cup for supporting the radioactive "seed." Following insertion of the needle pusher into the selected tissue mass, the radioactive "seed" is released by pressing the central needle downwardly relative to the surrounding outer tube, with the point of the needle ejecting the "seed" from the end plate or cup of the outer tube.

To percutaneously insert marker 30 in accordance with this first approach, the marker is positioned on the end of the needle pusher (in place of the radioactive "seed"), is forced through skin 402 and, using feedback from the imaging system, is guided to the region where it is desired to implant the marker. Then the marker 30 is ejected from the needle pusher by urging the central needle forwardly into the inner tube.

A second approach for implanting markers 30 involves creating a small, *e.g.*, 5-10 mm, incision (not shown) in the skin 402 overlying tissue portion 24. Next, a scalpel is inserted through the incision so as to form a slit in the underlying tissue portion extending to the position where it is desired to implant a maker 30. Then a marker 30 is inserted through the slit to such position using a tweezers, needle pusher, trocar or other suitable tool. Other markers 30 are implanted through separate incisions in skin 402 in similar manner so as to bracket tissue volume 22.

Referring now to Figures 1 and 12-14, a third approach for implanting markers 30 is to form a relatively large, *e.g.*, 1-3 cm, incision 404 (see Figure 12) in skin 402 overlying tissue mass 26. Next, incision 404 is pulled open as illustrated in Figure 14 using retractors or other conventional devices to form a relatively large open region 406 above tissue mass 26. Markers 30 are then implanted into tissue portion 24 using either the first or second approaches described above. Other approaches for implanting markers 30 so as to bracket tissue mass 26 are also encompassed. The speed and accuracy with which markers 30 may be implanted, and the trauma associated with implantation should be considered in selecting other approaches for implanting markers 30.

F. Marker Identification

Once tissue mass 26 has been bracketed or otherwise marked, tissue volume 22 can be removed. As described in more detail below, one procedure involves identifying the boundaries of tissue volume 22 using a probe 32 and detector 34 that is appropriate for the type of marker 30 used, as discussed above. Using information from detector 34 regarding such boundaries, tissue volume 22 is then removed using a scalpel or other tool, with tissue anchor 300 preferably, but not necessarily, being used to stabilize the tissue volume during removal. Another procedure is similar to the first, except that tissue anchor 300 is not used.

For both of these procedures, as the first step the surgeon typically identifies the boundaries of the tissue volume using system 20 or otherwise marks the location of the tissue mass 26 as described in more detail below. This step is generally needed because in practice markers 30 will often be implanted by another doctor, *e.g.*, a radiologist, as a separate procedure. The boundaries of tissue volume 22 are identified by moving probe 32 in the general region of the tissue volume and then monitoring the detection information (*e.g.*, sound, light, dial movement, image clarity and the like) provided by detector 34. As noted above, detector 34 may provide this information when probe 32 is moved within a predetermined proximity of a given marker 30, or may provide this information in a form that changes with changes in proximity of the probe to the marker (*e.g.*, a light gets brighter as the probe is moved toward a marker and dimmer as it is moved away).

The interaction between marker 30 and probe 32 and detector 34 depends upon the detection characteristic of the marker. In the case of marker 30a, which emits gamma radiation 40 (Figure 2a) on a continuous basis, a probe and detector of the type described in U.S. Patents Nos. 5,170,055 and 5,246,005 to Carroll et al. (the "C-TRAK probe"), as discussed above, may be satisfactorily used to detect the markers. The C-TRAK probe includes a radiation detector, *e.g.*, a scintillation crystal, which provides an output signal that is believed to vary as a function of the flux density of the gamma rays 40 emitted by marker 30a. Changes in this output signal are then converted into humanly recognizable detection information, *e.g.*, sound, having a characteristic, *e.g.*, pitch or tempo in the case of sound, that varies with changes in gamma ray flux density. By observing the location of probe 32 when the detection information from detector 34 indicates the probe is closest to a given marker 30a, the surgeon can mentally note where the marker is located. Repetition of this process will result in identification of the location of all markers 30a.

Referring to Figures 2b and 7, in the case of marker 30b, which generates a magnetic field 42, probe 32' and detector 34' are used to detect the marker. To locate a marker 30b, the surgeon moves probe 32' in the general region of tissue volume 22, with the result that as the probe approaches a given marker 30b its Hall effect sensor (not shown) generates an output signal having a voltage that increases as the probe is moved toward the marker. Similarly the voltage of the output signal decreases as probe 32' is moved away from the marker 30b. The output signal of probe 32' is provided via line 120 to amplifier 122, which amplifies the output signal from the probe. As discussed above, the amplified voltage signal from probe 32' is displayed on signal meter 126 and is also delivered to voltage controlled oscillator 128. The latter generates an oscillating signal, the frequency of which varies as a function of the voltage of the amplified signal provided to voltage controlled oscillator 128. This signal is then amplified by amplifier 130, and the amplified signal then drives speaker 116 such that the pitch of the sound provided by the speaker 116 varies as a function of proximity of probe 32' to marker 30b. By observing signal meter 126 and/or listening to speaker 116, the surgeon can assess when the probe 32' is positioned closest to a selected marker 30b. Repetition of this process will result in identification of the location of all of markers 30b.

Turning now to Figures 2c, 3a, 3b and 8, marker 30c, which generates an RF signal 44, is identified using probe 32" and detector 34" in the following manner. RF exciter 60 is operated so as to produce an RF exciter signal 46. More particularly, radio frequency generator 62 (Figure 3B) generates a radio frequency signal which is amplified by RF amplifier 64, following sensitivity adjustment using gain adjustment 66, with the amplified signal being provided to antenna 68 for transmission to markers 30c. RF exciter 60 is positioned sufficiently close to markers 30c that RF exciter signal 46 is received by antenna 52 of the markers and is of sufficient strength to drive radio frequency generator 56 of the markers. Following detection and regulation by circuit 54 (Figure 3A) of the signal 46 received by antenna 52, radio frequency generator 56 generates an RF signal which is transmitted by antenna 52 as RF signal 44. Each marker 30c can transmit RF signal 44 at a frequency that is unique to the marker, while an RF exciter signal 46 having a single frequency can be used for all of the markers 30c, with the frequency of signal 46 being different than the frequencies of signals 44.

Once exciter 60 has been activated so as to cause marker 30c to generate RF signal 44, detection of the marker commences. This is achieved by positioning probe 32" (Figure 8) on or adjacent skin 402 adjacent tissue volume 22, and then monitoring proximity information provided by analog signal strength display 150 and/or speaker 116 of detector 34". More specifically, following receipt of RF signal 44 by receive antenna 140 of probe 32", the signal is filtered by selectable notch filter 142 of probe 32". By correlating a given marker 30c, *e.g.*, marker $30c_1$, with a corresponding representation on the adjustment knob (not shown) that controls selectable notch filter 142, *e.g.*, the reference number "1," the surgeon can identify the location of the given marker. The knob for adjusting selectable notch filter 142 is then moved to a different position when detecting a second marker 30c, *e.g.*, marker $30c_2$.

Signals from receive antenna 140 that are passed through selectable notch filter 142 are then amplified by RF amplifier 144 with the adjustment of the amplifier gain being provided as needed using gain adjustment 146. The amplified signal is then provided to rectifier/integrator 148 where the signal is rectified and time filtered. The strength of signal 144 detected by detector 34" is then displayed via analog signal strength display 150 and is provided to voltage controlled oscillator 152. The latter creates an oscillating signal, the frequency of which varies as a function of the voltage of the signal provided by rectifier/integrator 148. The output signal from voltage controlled oscillator 152 is then amplified by audio amplifier 154 and delivered to drive speaker 116. The pitch of the sound provided by speaker 116 will vary as a function of the frequency of the signal provided by voltage controlled oscillator 152, and as an ultimate function of the proximity of probe 32" to a given marker 30c. By observing the location of probe 32" when the detection information from detector 34" indicates the probe is closest to a given marker 30c, the surgeon can mentally note where the marker is located. By repeating this process for each of the markers 30c with appropriate adjustment of selectable notch filter 142, all of the markers 30c may be located.

Referring to Figures 2d, 3a, 3b and 8, marker 30d may also be detected using detector 34" in substantially the same manner discussed above with respect to marker 30c. One significant difference, however, is the fact that RF exciter 60 (Figure 3B) is not used insofar as marker 30d contains its own power source.

Turning next to Figures 2e, 2f, and 4-6, for markers 30e and 30f, which are designed to provide high image contrast when imaged with ultrasound, probe 32 includes a conventional ultrasound transducer (not shown) that generates ultrasound in a conventional frequency range, *e.g.*, 7.5 MHz, and receives back reflection of the ultrasound signal. Detector 34 is the image processor and display (neither shown) of a conventional ultrasound apparatus which is connected to the ultrasound transducer. Markers 30e or 30f are identified by scanning the general region of tissue volume 22 with probe 32, and monitoring the ultrasound image of the markers provided by detector 34. This ultrasound image permits the surgeon to identify the placement of all of the markers, and hence the boundaries of tissue volume 22.

In the case of marker 30e, the latter is caused to vibrate at a frequency that is generally significantly less than that of the ultrasound generated by the ultrasound transducer in probe 32. This creates, through what is believed to be a Doppler shift phenomenon, enhanced image contrast in the ultrasound signal reflected off markers 30e. Vibration of a marker 30e is effected by operating RF exciter 92 so that radio frequency generator 94 generates a radio frequency signal which is amplified by amp 96 and then transmitted by antenna 100. Antenna 80 of marker 30e receives this RF signal, which is detected and regulated by circuit 84 so as to generate an oscillating electrical signal that is provided to piezoelectric device 86. This signal causes the piezoelectric device 86 to mechanically oscillate, which oscillations are transferred via support 88 to outer housing 90 of marker 30e, thereby causing the housing (and hence the marker) to vibrate.

G. Tissue Removal

Following identification of tissue volume 22 using the procedures outlined above, surgical removal of the tissue volume commences. Referring to Figures 12 and 14, the first of the two procedures for removing tissue volume 22 referenced above commences with the formation of an incision 404 (Figure 12) in skin 402 above tissue volume 22. The length of incision 404 is typically about equal to, or slightly greater than, the distance between two markers 30 lying on a given axis, *e.g.*, the Y-axis as illustrated in Figure 12. Next, portions of skin 402 adjacent incision 404 are pulled apart by retractors or other known devices, so as to form open region 406 (Figure 14) and expose tissue portion 24 beneath.

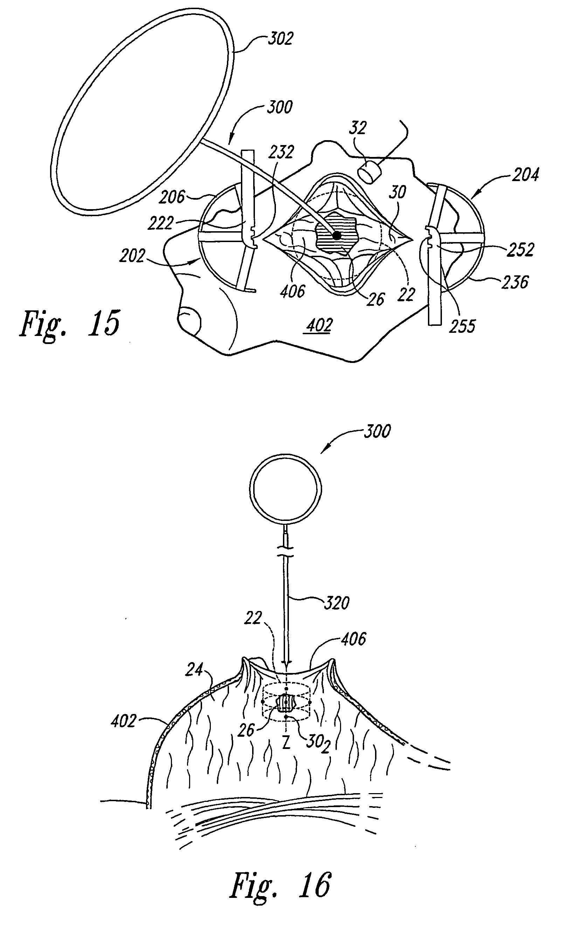

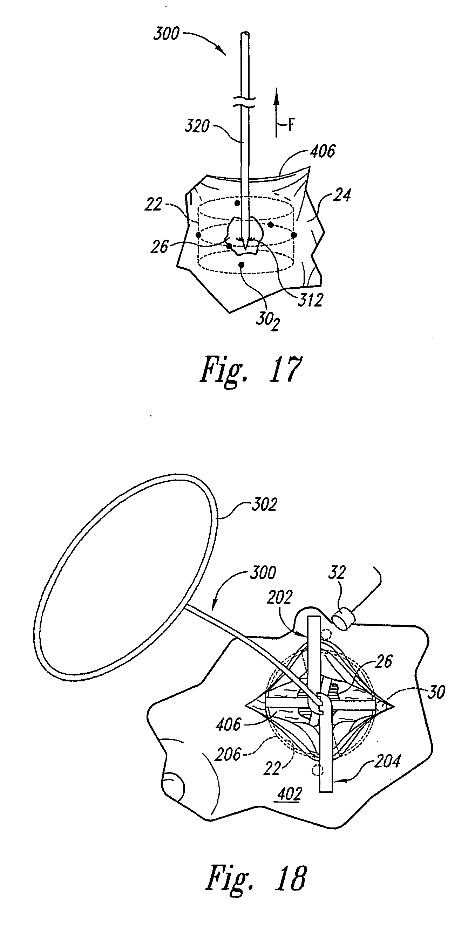

Referring now to Figures 9-11 and 15-17, as the next step, tissue anchor 300 is inserted in tissue mass 26 so as to assume the extended position illustrated in Figure 11. This is achieved by inserting a finger into ring 302, then pulling rod 304 upwardly (as illustrated in Figure 10) with respect to cannula 320 so that pin 308 moves in slot 328 toward the end thereof closest to proximal end 324 of the cannula. In this retracted position, cannula 320 is grasped and is inserted through open region 406 into tissue volume 22 so that its distal end 326 is positioned substantially in the center of tissue mass 26. This placement may be achieved under the guidance of an imaging system (not shown) that is capable of imaging tissue anchor 300, *e.g.*, ultrasound or X-ray imaging systems. Alternatively, using system 20, the location a marker 302 lying beneath tissue volume 22, as illustrated in Figures 16 and 17, is identified using the procedure described above to identify the tissue volume. By identifying the depth at which marker 302 is located and comparing this to the length of cannula 320 inserted into tissue volume 22, distal end 326 may be positioned centrally within tissue mass 26.

Next, ring 302, and hence rod 304 attached thereto, is forced downwardly (as viewed in Figure 15) relative to cannula 320 until pin 308 contacts the end of slot 328 closest to distal end 326. As rod 304 moves within cannula 320 toward this extended position, anchor members 310 are forced out through apertures 330 and into tissue mass 26 (see Figure 17). Then, ring 302, and hence rod 304, is rotated slightly so as to cause pin 308 to move into pocket 329.

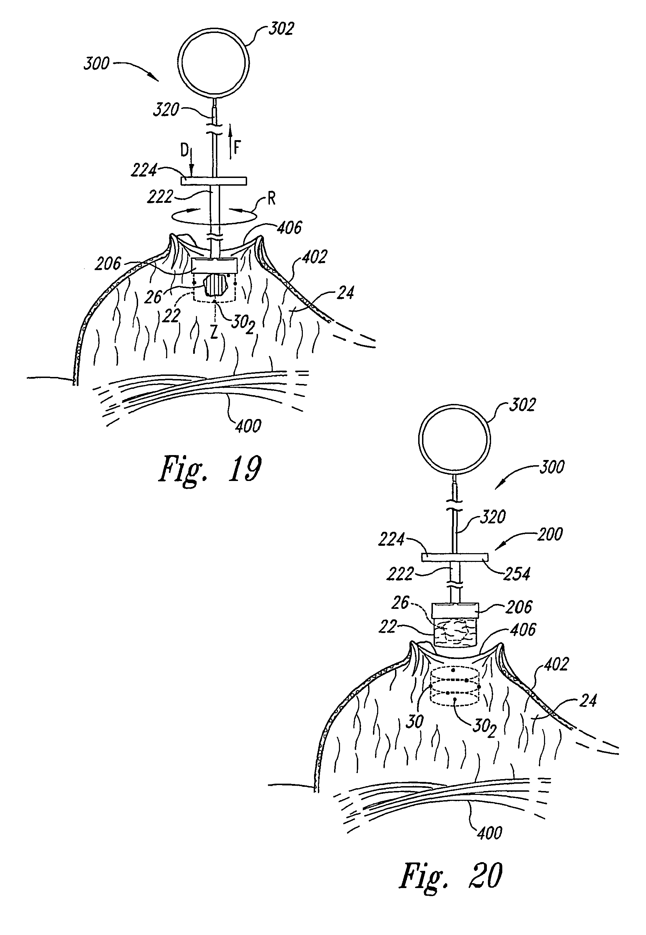

The next step in the removal of tissue volume 22 is assembly and placement of a cutter 200 in open region 406. Referring to Figures 15 and 18-20, the cutter 200 includes cutter portions 202 and 204 that can be positioned adjacent open region 406, as illustrated in Figure 15. Next, the cutter portion 202 is positioned in open region 406, and a curved plate 206 of the cutter portion 202 is inserted under portions of skin 402 adjacent the open region, as illustrated in Figure 18. Next, the cutter portion 204 is similarly positioned in open region 406. Then, cutter portions 202 and 204 are moved toward one another so that cannula 320 of tissue anchor 300 is received in an elongate groove 232 in a central handle section 222 and in an elongate groove 255 in a central handle section 252. Cutter portions 202 and 204 are moved even closer to one another so that central handle sections 222 and 252 engage one another. When positioned in this manner, ends of curved portion 206 of cutter portion 202 engage ends of curved portion 236 of cutter portion 204 so as to form a substantially continuous curved cutting edge. Also when positioned in this manner, a longitudinal axis of cutter 200 extends substantially parallel to the elongate axis of cannula 320, both of which are substantially co-axial with the Z-axis extending through tissue volume 22. (See Figures 16 and 19).

Next, the position of cutter 200 relative to markers 30 is determined by comparing the location of markers, which is typically determined by using probe 32 and detector 34 in the manner described above, to the position of the cutter. Then, the location of cutter 200 is adjusted so that the longitudinal axis of cutter 200 is substantially co-axial with the Z-axis of the tissue volume 22, as illustrated in Figure 19. In some cases the surgeon will recall the location of markers 30 from the prior marker identification step, and so it will be unnecessary to again locate the markers. However, when tissue portion 24 is amorphous and pliable, as is the case when breast tissue is involved, it is recommended that this alignment of cutter 200 with tissue portions 30 using probe 32 and detector 34 be performed before any cutting of tissue volume 22 commences.

In connection with the initial insertion of cutter 200 in open portion 406, an appropriately sized cutter 200 is selected such that the radius of curved plates 206 and 236, as measured radially outwardly from the longitudinal axis, is substantially the same as the radius of tissue volume 22 as measured radially outward from the Z-axis. While this relationship between the radii of curved plates 206 and 236 of cutter 200 and the radius of tissue volume 22, as measured with respect to Z-axis, is preferred, in some cases it may be satisfactory to use a cutter having a radius that is greater than or less than the radius of the tissue volume 22. Also, the height of curved portions 206 and 236 is another factor considered in selecting an appropriate cutter 200.

Referring to Figures 16-20, as the next step in the removal of tissue volume 22, ring 302 of tissue anchor 300 is typically pulled upwardly in the direction of arrow F (see Figures 17 and 19) sufficiently to tension tissue volume 22 and adjacent portions of tissue portion 24. By this tensioning of tissue volume 22 and tissue portion 24 the tendency of the tissue portion to compress under the force of a cutting device is reduced. Also, this tensioning of tissue volume 22 serves to stabilize the tissue volume during the surgical removal process.

In some cases, sufficient tissue stabilization can be achieved merely by holding tissue anchor 300 in a substantially fixed position relative to tissue volume 22. In other words, no force in the direction of arrow F is applied to tissue anchor 300 except as may be necessary to hold the tissue anchor in a stable position.

Then, while stabilizing tissue volume 22 with tissue anchor 300, preferably, but not necessarily by maintaining an upward force on the tissue anchor, the surgeon grips cutter 200 and begins pressing downwardly toward tissue volume 22, *i.e.*, in the direction of arrow D (see Figure 21). At the same time, the cutter is rotated about its longitudinal axis in either or both a clockwise and counterclockwise direction, *e.g.*, in the direction indicated by curved arrow R (see Figure 19). The elongate grooves 232 and 255 (Figure 15) are sized to permit cutter 200 to rotate relatively freely about cannula 320 positioned therein.

As cutter 200 is rotated about its longitudinal axis and is urged downwardly towards tissue volume 22, it cuts tissue volume 22 along its outer boundary. Progress in removing tissue volume 22 is generally periodically determined by comparing the position of curved plates 206 and 236 of cutter 200 relative to markers 30 using probe 32 and detector 34 to identify the locations of markers 30 and then comparing such locations with the location of the cutter. In particular, a determination can be made as to when tissue volume 22 has been severed from tissue portion 24 to a depth defined by marker 302 (Figure 21) defining the bottom or innermost portion of the tissue volume. Thus, by iteratively comparing the position of cutter 200 to the locations of markers 30 using marker location information acquired from detector 34 based on proximity information provided by the detector, a surgeon can determine when the cutting operation is completed and cutter 200 can be removed from tissue portion 24, as indicated in Figure 20.

Depending upon the size of cutter 200 relative to the placement of markers 30, the latter may remain in place in tissue portion 24 following removal of tissue volume 22, as indicated in Figure 20. If such as the case, markers 30 are then subsequently removed by first locating the markers using probe 32 and detector 34 and then removing the markers with a suitable instrument, *e.g.*, tweezers. In other cases, the markers will be included in the tissue volume 22.

In some cases, it will be necessary to sever the bottom or innermost portion of tissue volume 22 from tissue portion 24 so as to permit removal of the tissue volume. A scalpel or other conventional tool may be used to perform this final severing of the tissue volume. The precise location where this final incision is made may be determined by again locating the position of marker 302 using probe 32 and detector 34. By leaning tissue anchor 300 and cutter 200 to one side, a surgeon can typically follow the incision created by cutter 200 with a scalpel or other tool down to the region where marker 302 is located and tissue volume 22 remains attached to tissue portion 24.

As noted above, in some circumstances a marker 302 is not required when the bottom or innermost portion of tissue volume 22 is positioned immediately above a different type of tissue, *e.g.*, a pectoral muscle 400. In such case, the surgeon can assess when cutter 200 has been inserted sufficiently deep into tissue portion 24 by merely observing when bottom cutting edges of the cutter are about to engage the different type of tissue.

Referring to Figure 1A, by inserting markers 30 at staggered locations along the Z-axis, the relative depth of cutter 200 in tissue portion 24 can be determined by locating specific markers using probe 32 and detector 34. The location of such markers 30 is then compared with the location of cutter 200 to determine the depth of the cut. For example, if markers 30c are installed at positions $X_1$ and $X_2$ in Figure 1a, and each marker has a unique frequency, these markers can be uniquely identified by detector 34" (Figure 8) in the manner described above.

Referring to Figure 1B, by positioning more than four markers, *e.g.*, eight markers as illustrated in Figure 1B, the boundaries of tissue volume 22 can often be more readily defined during the removal of the tissue volume. This is so because increasing the number of markers 30 used increases the quantity of information received from detector 34 regarding the boundaries of tissue volume 22.

While the use of cutter 200 in connection with the removal tissue volume 22 often expedites removal of the tissue volume, many other cutters or instruments can be used to remove, treat, monitor, or otherwise perform some procedure on the tissue volume. In this regard, a conventional scalpel may often be satisfactorily employed in place of cutter 200. Also, under certain circumstances it may be desirable to initiate an incision with cutter 200, and then complete the incision with a scalpel. It will be appreciated that other types of cutters and systems for manipulating the tissue can be used, such as using a vacuum to pull up on the tissue, extending an "umbrella" at the end of a stabilizer to pull up on the tissue, vibrating the cutter to cut the tissue (either in lieu of or in addition to rotating the cutter, and using rotational electrocautery).

The process of removing tissue volume 22 using a scalpel also preferably commences by inserting tissue anchor 300 in tissue volume 22 in the manner described above. The location of markers 30 are also determined prior to and during the removal of tissue volume 22 by scalpel in the manner described above. Thus, during the removal of tissue volume 22, the boundaries thereof may be repeatedly identified by locating markers 30 using probe 32 and detector 34. As noted above, it is generally advantageous to use tissue anchor 300 when removing tissue volume 22 with a scalpel because by stabilizing the tissue volume, and surrounding regions of tissue portion 24, it is easier to maintain alignment of the scalpel with the boundaries of the tissue volume. However, it is to be appreciated that the use of tissue anchor 300 is a preferred, but not essential, aspect of the present method of bracketing and removing tissue volume 22.

Referring now to Figure 2g and Figure 13, provided for comparison purposes, as noted above, probe 32 and detector 34 are not used in connection with marker 30g. The detection characteristic of markers 30g is the release of a colored dye 78 in surgical cavity adjacent the markers. In an alternative example, the markers can be capsules that each can have a different color, and the colored markers can be implanted in a manner to define the desired margin for guidance during a percutaneous biopsy procedure, excisional procedures, and other procedures. Removal of a tissue volume 22 bracketed by markers 30g differs from the removal of tissue volume when bracketed by the other embodiments of marker 30 in that the location of marker 3Dg is not determined by the surgeon prior to initiation of the removal of tissue volume 22. Practically speaking, this is more a difference in the process for removing tissue volume 22 than a difference in the composition and construction of marker 30g. This is so because for implantation purposes, marker 30g must necessarily be imageable by some form of imaging system, which imaging system could, in most cases, also be used by the surgeon to identify the location of marker 30g prior to and in connection with the removal of tissue volume 22. For example, if marker 30g is initially implanted by imaging the marker using an ultrasound system, then marker 30g is actually a marker 30f. Thus, in connection with the following description of the process of removing tissue volume 22 bracketed with markers 30g, it is assumed the markers are not located by the surgeon prior to, or in connection with, the removal of tissue volume other than by visual observation, as discussed below.

Removal of tissue volume 22 bracketed by markers 30g also preferably commences by installing tissue anchor 300 as described above. Again, the use of tissue anchor 300 is preferred, but not mandatory. Next, the surgeon commences cutting the general region of tissue volume 22, which can be defined by colored marks, Kopanz needles or other known techniques. Then, the removal of tissue volume 22 proceeds using either cutter 200, or a scalpel or other cutting device. As this removal of tissue volume 22 is performed, tissue anchor 300, if used, is manipulated to stabilize tissue volume 22 in the manner described above. As cutter 200, the scalpel or other cutting device (*e.g.*, a vacuum assisted cutting device) encounters a marker 30g, the capsule of the marker is severed releasing the colored dye 78. This advises the surgeon that a boundary of tissue volume 22 has been encountered. It may be advantageous to use a given color of dye in markers 30g defining one side of the boundary of tissue volume 22, while the markers 30g defining an opposite side include a different color of dye. By defining the boundary of tissue volume 22 with a sufficient number, *e.g.*, 10-25, of markers 30g, the boundary of tissue volume 22 can typically be identified by iteratively cutting and observing whether dye appears in the surgical cavity.

As noted above, markers 30a-30f may all include colored dye 78 within an outer capsule that is sufficiently tough to withstand insertion and yet is relatively easily cut by cutter 200, a scalpel or other cutting device. Such use of dye in markers 30 provides another source of information for the surgeon regarding the boundary of tissue volume 22.

One advantage of certain tissue bracketing systems 20 is that they permit the relatively precise identification of the boundaries of tissue volume 22 without the need for needles, wires or other cumbersome apparatus projecting from tissue portion 24. As such, bracketing system 20 permits a surgeon to relatively quickly and easily identify the tissue boundary of tissue volume 22 and remove the tissue volume. In addition, system 20 is ideally adapted for bracketing a tissue volume 22 in amorphous, pliable tissue, such as breast tissue.

Another advantage of certain cutters 200 is that they permit a tissue volume 22 of relatively large diameter to be removed through a relatively small incision 404 or percutaneously. This advantage is useful in this era when tissue-conserving therapies are being emphasized.

By stabilizing tissue volume 22 using tissue anchor 300 of the present invention, the accuracy with which a surgeon can remove tissue volume 22 is also enhanced compared to techniques that do not use a tissue stabilizer or anchor. Also, the accuracy of removing tissue may be further enhanced by docking the tissue stabilizer or anchor to the first implanted tissue marker by using a first marker in the tissue stabilizer and the position detection system. This advantage of the present embodiment arises because tensioning of the tissue volume 22 by pulling upwardly on tissue anchor 300 serves to retain the tissue portion in a relatively stable position. Indeed, even holding tissue anchor 300 in a substantially fixed position relative to the tissue volume 22 with which it is engaged typically provides beneficial stabilization of the tissue volume.

While cutter 200 and tissue anchor 300 may be advantageously employed in connection with the present method of bracketing and removing tissue volume 22, it is to be appreciated that the cutter and tissue anchor have application in many other contexts. More specifically, in any application in which it is desired to remove a volume of tissue through as small an incision as possible, cutter 200 has utility. Similarly, when it is desired to stabilize a piece of tissue in connection with surgical removal or other treatment of the piece of tissue, whether or not within the bracketing context of the present invention, tissue anchor 300 also has important application. Likewise, the system of bracketing a tissue mass is also useful in other applications, such as radiation therapy, and in connection with other body parts.

II. Alternate Systems and Methods for Locating, Monitoring and/or Treating Target Locations Within a Human Body

A. Overview of System Components and Operation

Figure 21 is an isometric view of a system 1000 for locating a target location T within a human body H. The target location T shown in Figure 21 can be a lesion, tumor, or other area of interest on or within a soft tissue region (*e.g.*, a breast "*B*"), an organ, the colon, a bone structure, or another body part. The particular components of the system 1000 are best understood in light of the relationship between the components and the operation of the system. Therefore, the following description will initially explain an overview of the components and the general operation of the system 1000.

The system 1000 includes a wireless implantable marker 1100, an instrument 1120, a position detection system 1200, and a user interface 1300. The wireless implantable marker 1100 can be implanted at a precise location with respect to the target location 1000 using stereotactic imaging systems and other procedures known in the art as explained above. In operation, the position detection system 1200 determines the location of the wireless implantable marker 1100 and the location of the instrument 1120 relative to a reference location to determine the relative position between the target location T and the instrument 1120. The position detection system 1200 is coupled to the user interface 1300 to convey the relative position between the target location 1000 and the instrument 1120 in a manner that allows a surgeon to intuitively understand the position and the orientation of the instrument 1120 relative to the target location 1000 without additional imaging equipment. As a result, the system 1000 is particularly useful for applications in which the patient cannot immediately proceed from an imaging procedure to another procedure, or when intraoperative imaging is not practical or economical.

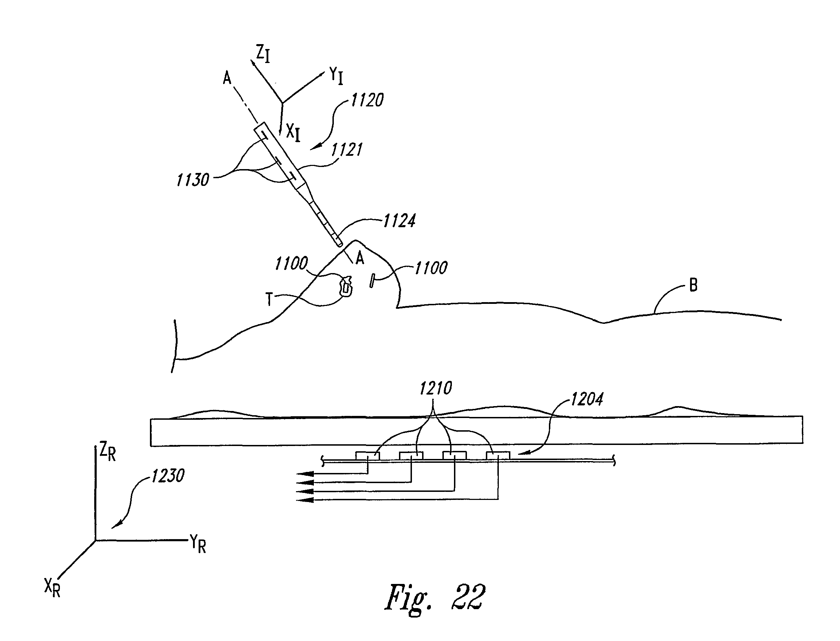

Figure 22 is an elevational view illustrating selected wireless implantable markers 1100, the instrument 1120, and a portion of the position detection system 1200 in greater detail. The wireless implantable marker 1100 can be one of the markers described above with reference to Figures 1-20. Alternatively, the wireless implantable marker 1100 can be a resonating marker or another type of marker as described below in more detail with reference to Figures 23A-33. In general, at least one wireless implantable marker 1100 is implanted at a location relative to the target location 1000. As shown in Figure 22, one wireless implantable marker 1100 is implanted within the target location T and another wireless implantable marker 1100 is implanted adjacent to the target location T. The wireless implantable markers 1100 emit in several examples a response energy in reaction to an excitation energy emitted by the position detection system 1200. The position detection system 1200 can sense the intensity of the response energy and determine the location of the individual implantable markers 1100 relative to a reference location.

This implementation could be used with a device that is at a known location relative to the position detection system reference location. For example, an external beam radiation could be applied to a target location defined by the first implantable marker or otherwise monitored when the position of the beam applicator is known relative to the reference location of the position detection system. A suitable external beam radiation device is the PRIMIS Linear Accelerator from Siemens Medical of Concord, CA.

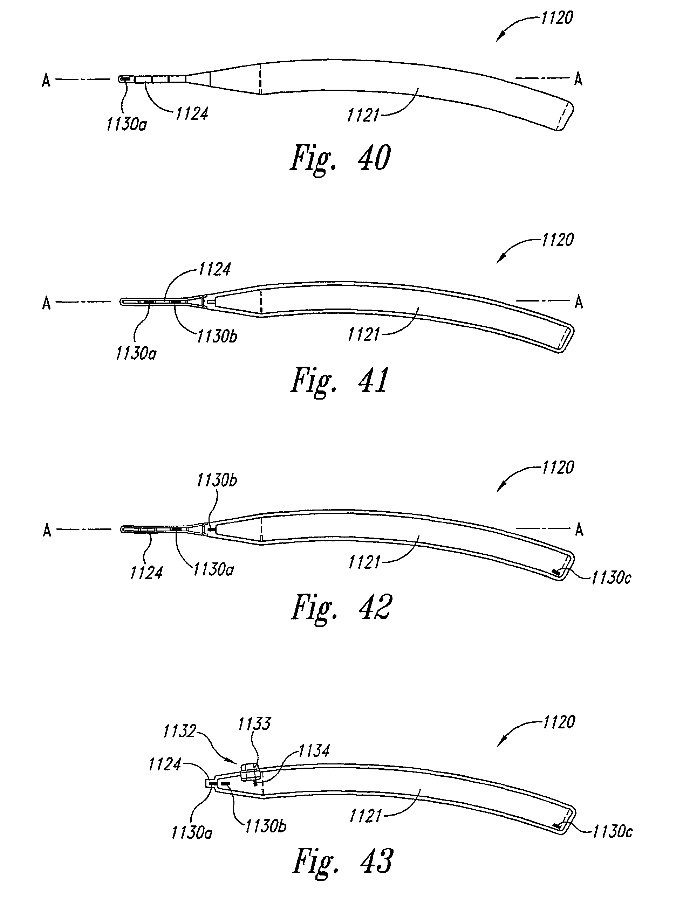

The instrument 1120 can include a handle 1121, a function-site 1124 coupled to the handle 1121, and at least one instrument marker 1130. The function-site 1124 can be a tip of the instrument 1120 or a portion of the instrument 1120 that cuts, ablates, deposits, images or otherwise treats or monitors the target location T. Several of various types of instruments with different function-sites are described in more detail below with reference to Figures 40-52. The instrument markers 1130 can be the same type of wireless markers as the implantable marker 1100, or alternatively the instrument markers 1130 can be a different type of wireless marker. The instrument markers 1130 can also be "wired" markers that are directly coupled to the position detection system 1200. The position detection system 1200 can also gauge the instrument markers 1130 to determine the position of the instrument relative to a reference location.

As shown in Figure 22, the instrument 1120 includes three instrument markers 1130 including two instrument markers 1130 that are attached to the instrument 1120 along an alignment axis A-A, and a third instrument marker 1130 that is offset from the alignment axis A-A. By knowing the distance between the function-site 1124 and the array of instrument markers 1130, the position detection system 1200 can determine the position and the orientation of the function-site 1124 based upon the positions of the three instrument markers 1130.

Referring to Figures 21 and 22 together, the position detection system 1200 (Figure 21) can include a processor 1202 (Figure 21), a detection array 1204 having a plurality of sensors 1210, and a transmitter 1220 (Figure 21). The transmitter 1220 can emit an excitation energy that causes the implantable markers 1100 to emit a response energy. Each sensor 1210 can include three coils arranged orthogonally around a magnetic core to measure the response energy emitted from the implantable markers 1100 and instrument 1130. The processor 1202 calculates the distance between each sensor 1210 and each of the markers 1100 and 1130 based upon the intensity of the response energy measured by the sensors 1210. The processor 1202 also correlates the distance measurements between each of the markers 1100 and 1130 to determine the individual locations of the markers 1100 and 1130 relative to a reference location 1230 (*e.g.*, a reference coordinate system). Based upon this data, the processor 1202 and/or another processor of the user interface 1300 can determine the relative position between the function-site 1124 of the instrument 1120 and the target location T. Suitable position detection systems 1200 and resonating signal elements that can be adapted for use with the implantable markers 1100 and/or the instrument markers 1130 are available from Polhemus, Inc. of Burlington, Vermont.

B. Wireless Markers

Figure 23A is a cut-away isometric view of a resonating marker that can be used for the implantable markers 1100 and/or the instrument markers 1130. In this example the resonating marker includes a casing 1140 composed of a biocompatible material, a signal element 1150 within the casing 1140, and a fastener 1160. The biocompatible material of the casing 1140 can be a suitable polymeric material, medical grade epoxy, metal, or other compound that can reside within a human body for a period of time. The signal element 1150 can be a resonating circuit that includes a magnetic core 1152, a coil 1154 wrapped around the magnetic core 1152, and a capacitor 1156 connected to the coil 1154. The core 1152 may be a magnetically permeable material, such as a ferrite. The signal element 1150 emits a response signal in reaction to an excitation energy at the resonate frequency of the circuit. As explained above, the excitation energy can be generated by the transmitter 1220 (Figure 21) of the position detection system 1200. In other examples, the signal element 1150 can be a mechanical resonator (*e.g.*, piezoelectric actuator), an RF emitter, a fluorescent material, a bipolar semiconductor magnet, or another suitable device or material that emits a response signal in reaction to an excitation energy. The fastener 1160 can have several different examples. In this particular example, the fastener 1160 is a shape-memory material that is straight in a stored position and coils to form a loop in a deployed position. The shape-memory material can be a spring, or it can be a substance that is straight at room temperature and coils at body temperature.

Figure 23B is an isometric cut-away view of another resonating marker. In this example, the resonating marker includes a biocompatible casing 1140 and a signal element 1150a. The marker can also include a fastener (not shown in Figure 23B). The signal element 1150a has three resonating members 1151a-c arranged orthogonally with respect to each other. The resonating members 1151a-c can also be configured in a non-orthogonal arrangement or any other suitable arrangement. Additionally, the signal element 1150a can include two or more resonating members such that this embodiment of the resonating marker is not limited to having three resonating members 1151a-c. Each resonating member 1151a-c can have a ferrite core 1152, a coil 1154 wrapped around the core 1152, and a capacitor 1156 coupled to each coil 1154. Each resonating member 1151a-c can be tuned to resonate at the same frequency or at different frequencies. When the resonating members 1154a-c resonate at different frequencies, this embodiment of a resonating marker can thus provide three different signals from a single marker so that the position detection system can detect not only the point position of the marker (*e.g.*, an X-Y-Z LOCATION), but also the pitch, roll and yaw of the marker relative to a coordinate system.

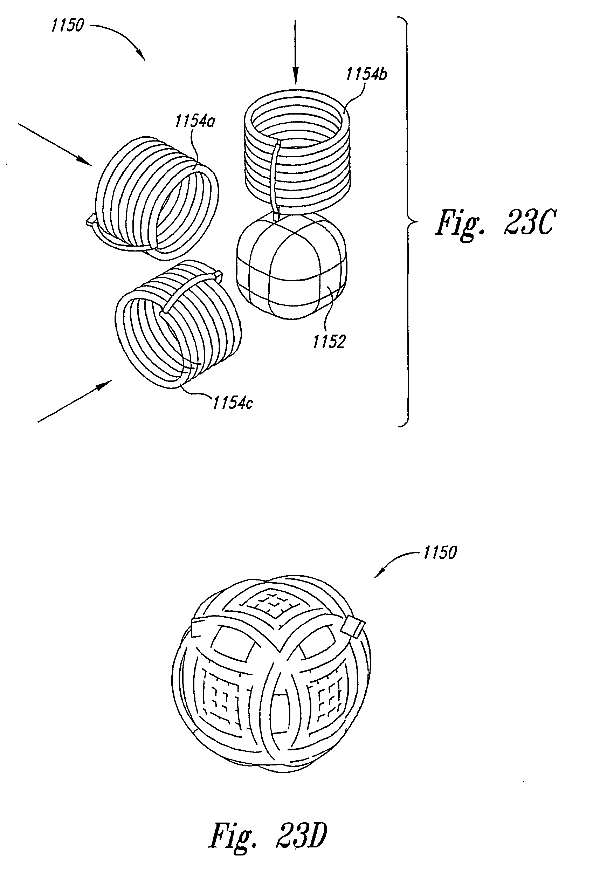

Figures 23C and 23D illustrate a resonating marker. In this example, the resonating marker has a single core 1152 and three coils 1154a-c. Each coil 1154a-c can be coupled to a capacitor (not shown), and each coil 1154a-c can generate a different signal. As such, this marker can be located in a manner similar to the marker described above with reference to Figure 23B. The core 1152 can accordingly be a ferrite block, and the coils 1154a-c can be wrapped around the block orthogonally to each other as shown in Figure 23D.

The resonating markers shown in Figure 23A and 23B are particularly useful because they can remain within a human body for a long period of time. These resonating markers can also have frequencies that are useful in applications in which a plurality of wireless markers 1100 are implanted. In such situations, it may be necessary to distinguish the implanted markers from one another. By using resonating markers that resonate at different frequencies, the position detection system 1200 can identify the "signature" of each marker by its unique frequency. A surgeon, therefore, can easily identify the relative location between a particular implanted marker 1100 and an instrument 1120.

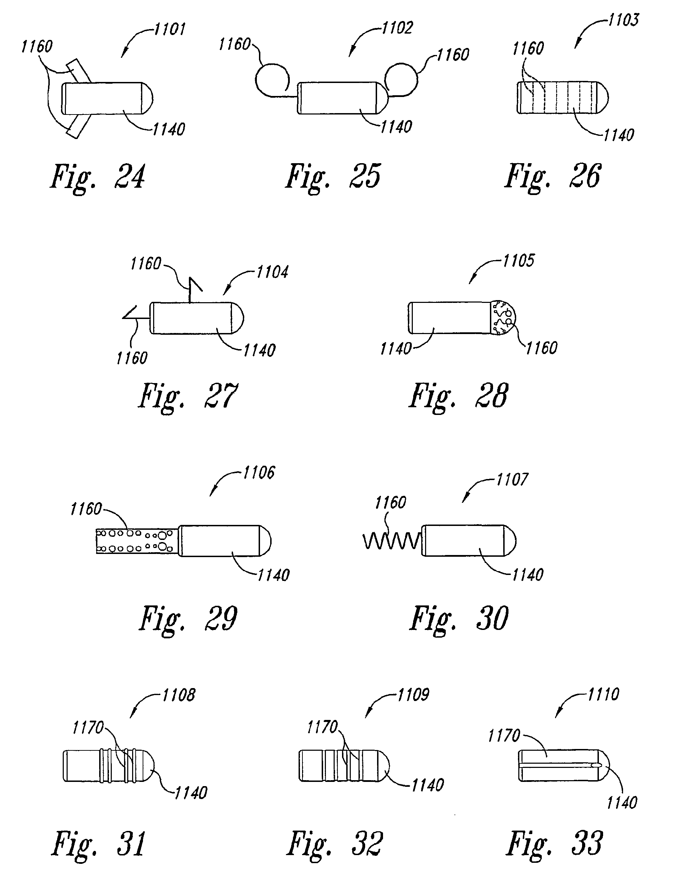

Figures 24-30 are side elevation views of several implantable markers 1101-1107. Each implantable marker 1101-1107 shown in Figures 24-30 has a biocompatible casing 1140. Additionally, the implantable markers 1101-1107 can also include a signal element 1150 or 1150a for emitting a resonating signal, such as a magnetic resonator, a mechanical resonator (*e.g.*, a piezoelectric actuator), an RF emitter, a magnet, a fluorescent material, or other suitable elements that can emit a signal for detection by the position detection system 1200 (Figure 21).

The implantable markers 1101-1107 have different types of fasteners 1160. The implantable marker 1101 shown in Figure 24 includes a fastener 1160 defined by legs that project away from the casing 1140 in the deployed position. The legs can be molded projections of the casing 1140, or the legs can be small springs that are biased to project away from the casing 1140. The implantable marker 1102 shown in Figure 25 includes a fastener 1160 defined by shape-memory loops on both ends of the casing 1140. In Figure 26, the implantable marker 1103 has a fastener 1160 defined by a surface texture, such as scales, that project away from the casing 1140. The surface texture of the implantable marker 1103 can be integrally formed with the casing 1140. Referring to Figure 27, the implantable marker 1104 can include a fastener 1160 defined by one or more barbs or hooks. Referring to Figures 28 and 29, the implantable markers 1105 and 1106 have fasteners 1160 defined by a perforated material through which tissue can grow, such as a mesh. The implantable marker 1105 shown in Figure 28 has a perforated tip, and the implantable marker 1106 shown in Figure 29 has a perforated tail. Referring to Figure 30, the implantable marker 1107 includes a fastener 1160 defined by a spring or a serpentine element extending from the rear of the casing 1140. It will be appreciated that the fasteners 1160 can have different configurations than the particular types of fasteners 1160 shown in Figures 24-30.

Figures 31-33 are side elevation views of several examples of implantable markers 1108-1110. The implantable markers 1108-1110 can include the biocompatible casing 1140 for implantation into a human body. The implantable markers 1108-1110 also include at least one identifier 1170 that is on and/or in the casing 1140. The identifier 1170 can be a radiopaque material that reflects radiation energy, an echogenic material that reflects ultrasound energy, and/or a groove or channel in the casing 1140 that can be observed by an imaging system. Alternatively, the identifiers 1170 can be a color or other marking that is visually distinguishable for viewing with a human eye. The identifiers 1170 provide another feature for distinguishing one marker from another that can be used in addition to, or in lieu of, using signal elements 1150 that emit different frequencies. The implantable markers 1108-1110 can also include fasteners 1160 as described above with reference to Figures 24-30, and/or signal elements 1150 or 1150a as described above with reference to Figures 23A and 23B.

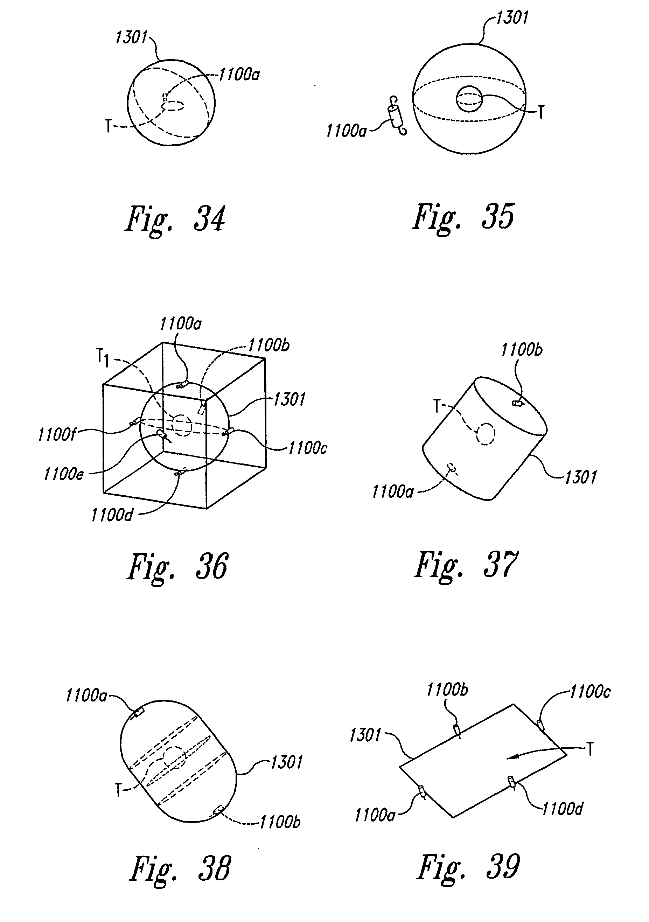

Figures 34 and 35 are isometric views of arrangements for implanting the wireless implantable markers 1100 relative to the target location T. Figure 34 illustrates an example in which only a first wireless implantable marker 1100a is implanted in the target location T, and Figure 35 shows an example in which only the first wireless implantable marker 1100a is implanted adjacent to or otherwise outside of the target location T. In either example, the location of the implantable marker 1100a relative to the target location T is determined when the marker 1100a is implanted or at another imaging procedure so that the marker 1100a provides a reference point for locating the target location T in subsequent procedures. The user interface 1300 can electronically generate a virtual margin 1301 relative to the target location based upon parameters defined by the physician and the location of the implantable marker 1100a. In other examples, it is not necessary to generate the virtual margin 1301 relative to the target location T. The physician can determine the shape of the virtual margin 1301 so that it defines a boundary for performing a particular procedure at the target location T. The virtual margin 1301 is typically configured so that it defines the desired boundary for the particular procedure at the target location T without unduly affecting adjacent areas. In the case of a lesion in a soft tissue region, for example, the physician can define a virtual margin 1301 that encompasses the lesion and an appropriately sized safety zone around the lesion that mitigates collateral damage to tissue proximate to the lesion. The virtual margin 1301 can be spherical as shown in Figures 34 and 35, or it can have any desired shape including rectilinear shapes, oval shapes, or compound shapes.

Figures 36-39 are isometric views of additional arrangements for implanting the wireless implantable markers 1100 relative to the target location T. Figure 36 illustrates an example in which six individual implantable markers 1100a-1100f are implanted in pairs along three orthogonal axes to define an excision boundary or another type of margin around the target location T. Figure 37 shows an example in which two individual implantable markers 1100a and 1100b define a cylindrical margin around the target location T. Figure 38 illustrates an example in which individual implantable markers 1100a and 1100b define an ovoid margin around the target location T, and Figure 39 illustrates an example in which four implantable markers 1100a-1100d define a rectilinear margin around the target location T. The individual implantable markers 1100a-1100f can define an actual margin by bracketing the target location T, or the positions of one or more of the individual markers 1100a-1100f can be used to generate a virtual margin 1301 for use with the user interface. Additionally, it will be appreciated that other arrangements for implanting the implantable markers 1100 and other types of margins can be used depending upon the particular procedure, the type of body part, and the shape of the target location T.

C. Instruments

Figures 40-42 are cut-away side elevation views of instruments 1120. The instruments 1120 include the handle 1121, the function-site 1124 coupled to the handle 1121, and at least one instrument marker 1130. The position detection system 1200 (Figure 1) can determine the position of the instrument markers 1130 relative to a reference location. Referring to Figure 40, this example of the instrument 1120 includes a single instrument marker 1130a at a predetermined location relative to the function-site 1124. The example of the instrument 1120 shown in Figure 40 provides at least a single position point for tracking by the position detection system 1200. When the instrument marker 1130 is a single-axis marker, such as the marker shown in Figure 23A, the instrument 1120 can be displayed as a single point by the user interface 1300 (Figure 1). The orientation of this particular example of the instrument 1120 cannot be displayed by the user interface 1300 because the single-axis marker does not provide sufficient data to determine the angle of the alignment axis A-A relative to a plane through the target location T (Figure 1) or the rotational position of the instrument 1120 around the alignment axis A-A. It may be possible, though, to have a single instrument marker 1130 define the location and orientation of the instrument 1120 if the position detection system 1200 and the instrument marker 1130 are sensitive enough to pinpoint the location and orientation of the single instrument marker 1130. For example, the multiple-axis markerS shown in Figures 23B-D are expected to provide sufficient data to define the location and orientation of the instrument 1120 using a single marker.

Figure 41 illustrates another example of the instrument 1120 having a first instrument marker 1130a and a second instrument marker 1130b. The first instrument marker 1130a is positioned at a first predetermined location relative to the function-site 1124, and the second instrument marker 1130b is positioned at a second predetermined location relative to the function-site 1124. The first and second instrument markers 1130a and 1130b can be positioned along the alignment axis A-A as shown in Figure 41, or at least one of the markers 1130a or 1130b can be offset from the alignment axis A-A. The example of the instrument 1120 shown in Figure 41 accordingly provides two position points that the position detection system 1200 can track. As a result, the position detection system 1200 can determine the angle of the alignment axis A-A relative to a reference plane so that the user interface 1300 can display the instrument 1120 as (a) a vector of varying length when the alignment axis A-A is not normal to the reference plane, or (b) as a point when the alignment axis A-A is at least approximately normal to the reference plane. When the instrument markers 1130a and 1130b are multiple-axis markers, the rotational orientation of the instrument 1120 relative to the alignment axis A-A can be determined such that both the position of the function-site 1124 and the orientation of the instrument 1120 can be displayed by the user interface 1300.

Figure 42 illustrates yet another example of the instrument 1120 having a first instrument marker 1130a, a second instrument marker 1130b, and a third instrument marker 1130c. The first and second instrument markers 1130a and 1130b can be positioned along the alignment axis A-A, but the third instrument marker 1130c is offset from the alignment axis A-A. This example of the instrument 1120 provides three position points for tracking by the position detection system 1200. As a result, the position detection system 1200 can determine (a) the angle of the alignment axis A-A relative to a reference plane, and (b) the rotational orientation of the instrument 1120 around the alignment axis A-A. The example of the instrument 1120 shown in Figure 42 accordingly permits the user interface 1300 to show the angle of the function-site 1124 relative to a reference plane, and the orientation of a leading edge of the function-site 1124 relative to the motion of the instrument 1120.

Figure 43 is a side elevational view of an example of the instrument 1120 including a wireless control 1132 for controlling an aspect of (a) the instrument 1120, (b) the position detection system 1200, and/or (c) the user interface 1300. The instrument 1120 shown in Figure 43 has three instrument markers 1130a-c, but will be appreciated that the instrument 1120 can have any of one or more instrument markers 1130. The wireless control 1132 includes an actuator 1133 and a transmitter 1134 coupled to the actuator 1133. The transmitter 1134 transmits or otherwise emits a signal indicating a control parameter. The transmitter 1134, for example, can be another marker that the position detection system 1200 can track. In one particular example, the transmitter 1134 is a resonating magnetic marker having a signal element 1150 as set forth above with respect to Figure 23. One advantage of using a resonating marker for the transmitter 1134 is that the system 1000 (Figure 21) can be controlled by a wireless instrument 1120 using the position detection system 1200 without additional types of receivers (*e.g.*, RF systems) that add to the complexity and cost of the system 100. Alternatively, the transmitter 1134 can be an RF device, a mechanical resonator, a permanent magnet, or another type of device that emits a frequency or another form of energy. When the transmitter 1134 is a marker, the position detection system 1200 detects the position of the transmitter 1134 and generates a control signal according to the position of the transmitter 1134.

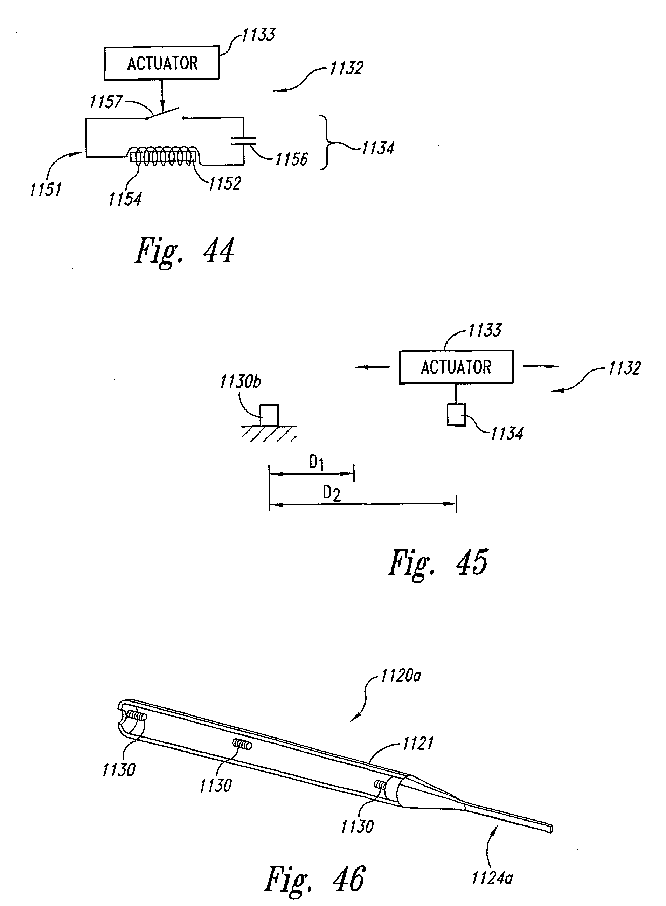

Figure 44 is a schematic view of one example of the wireless control 1132. In this example, the transmitter 1134 of the wireless control 1132 is a resonating marker having a resonating signal element 1150b similar to one of the signal elements 1150 or 1150a shown above in Figures 23A or 23B. The signal element 1150b includes a ferrite core 1152, a coil 1154 wrapped around the core 1152, a capacitor 1156 coupled to the coil 1154, and a cut-off switch 1157 between the coil 1154 and the capacitor 1156. The actuator 1133 can be a push-button coupled to the cut-off switch 1157 that breaks the circuit to deactivate the signal element 1150b. In operation, the physician can press the actuator 1133 to close the cut-off switch 1157 so that the signal element 1150b emits a resonating signal. The position detection system 1200 detects the signal from the signal element 1150b and generates a control signal that changes a parameter of the system 1000. The position detection system 1200, for example, can send a message to the user interface 1300 to change a display of the user interface 1300 to show the relative position between the instrument 1120 and one of several implanted markers 1100. This is particularly useful when a plurality of markers 1100 are implanted, such as the implanted markers 1100a-f in Figure 36, and the physician needs to know the position relative to a particular marker. In one example, the control 1132 can be used to cycle through the various markers 1100a-f by depressing the actuator 1133 to move from one marker to the next. The wireless control 1132 can also have several other applications that allow the position detection system 1200 to control other aspects of the system 1000 based upon input at the instrument 1120 .

Figure 45 is a schematic view of another example of the wireless control 1132. In this embodiment, the actuator 1133 is a slider mechanism that moves along the handle 1121, and the transmitter 1134 is another marker that can be detected by the position detection system 1200. The actuator 1133, for example, can be a linear slider or a rotational slider that has "click-stops" to indicate various control positions. In operation, the relative distance between the transmitter 1134 and a fixed marker attached to the instrument (*e.g.*, the second instrument marker 1130b) is determined by the position detection system 1200. A parameter of the instrument 1120, the position detection system 1200, and/or the user interface 1300 can be controlled according to the relative distance between the transmitter 1134 and the fixed marker. For example, if the distance between the transmitter 1134 and the second instrument marker 1130b is $D_1$, the user interface 1300 may display the distance between the function-site 1124 of the instrument 1120 and a first implanted marker. Similarly, if the distance between the transmitter 1134 and the second instrument marker 1130b is $D_2$, the user interface 1300 may display the relative distance between the function-site 1124 and a second implanted marker.

Figures 46-52 illustrate several instruments 1120a-g in accordance with various example of the invention. The instruments 1120a-g can each include a handle 1121, a function-site 1124 coupled to the handle 1121, and at least one instrument marker 1130 similar to the instruments 1120 described above with reference to Figures 40-42. The instruments 1120a-g can also include a wireless control similar to the wireless controls 1132 described above with reference to Figures 43-45. The differences between the instruments 1120a-g is generally the type of function-site 1124.

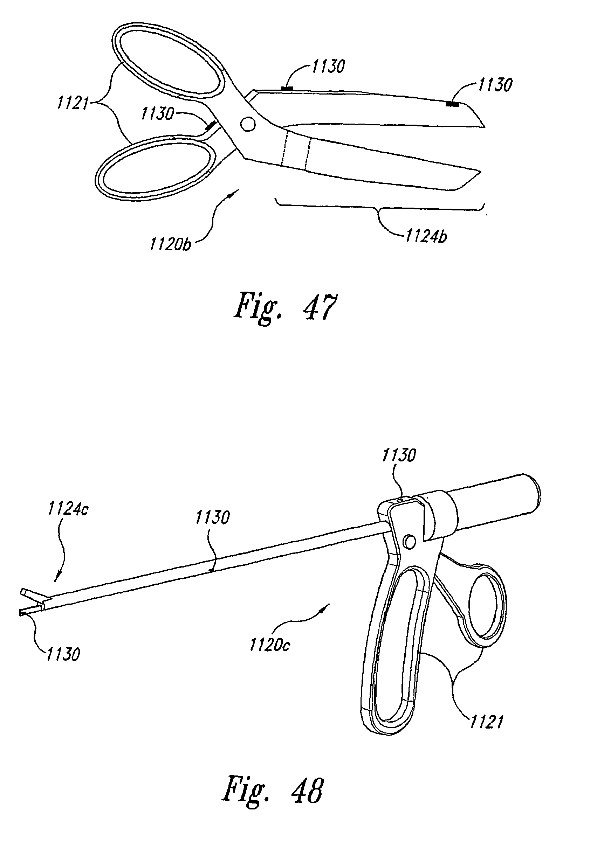

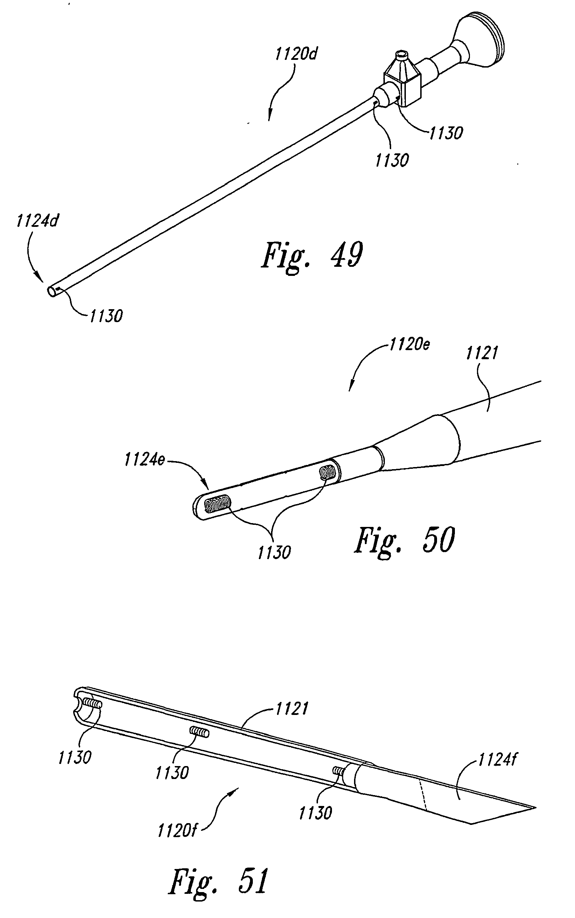

Figure 46 illustrates a smart Bovie 1124a that has a function-site 1124a defined by an RF cutting blade. Suitable RF cutting devices without the instrument markers 1130 are available from Valley Lab of Boulder, Colorado, under the part number E2516 Reusable Electrosurgical Pencil. Figure 47 illustrates a scissors 1120b that has a function-site 1124b defined by the cutting blades. Figure 48 illustrates a harmonic scalpel 1120c having a function-site 1124c defined by a harmonic cutting tip. Suitable harmonic scalpels without the instrument markers 1130 are available from Ethicon Endo Surgery of Cincinnati, Ohio, under the part name ULTRACISION HARMONIC SCALPEL®. Figure 49 illustrates a laproscope 1120d having a function-site 1124d defined by a distal end of the laproscope. Suitable laproscopes without the instrument markers 1130 are available from US Surgical of Norwalk, Connecticut, under the part name SURGIVIEV1I® Multi-Use Disposable Laproscope. Figure 50 illustrates a robotic probe 1120e having a function-site 1124e defined by a distal tip of the probe 1120e. The probe 1120e can be used to mark reference fiducials just prior to a surgical procedure to map out a desired cutting path. Figure 51 illustrates a scalpel 1120f having a function-site 1124f defined by a cutting blade. Suitable scalpels without instrument markers 1130 are available from Bard-Parker of Franklin Lake, New Jersey, such as single-use Scalpel No. 11.

Figures 52-59 illustrate several embodiments of tissue anchors in accordance with various embodiments of the invention. Figure 52 is an isometric view of one such tissue anchor 1550 and Figure 53 is a side view of the tissue anchor 1550 of Figure 52. In the tissue anchor 300 shown in Figures 9-11, the cannula 320 has a sharpened distal tip 326 and a plurality of apertures 330 through which individual anchor members 312 may pass. In the tissue anchor 1550 of Figures 52 and 53, though, the cannula 1552 has an open distal tip defining a single aperture through which the lead 1560 may pass. To facilitate passage of the tissue anchor 1550 through tissue to a desired anchoring site in the patient's body, the lead 1560 may be provided with a sharp distal tip 1565. The lead 1560 carries adjacent its distal end a plurality of anchor members 1566 similar to the anchor members illustrated in Figures 9-11. The proximal end of the cannula 1552 is provided with a manually graspable handle 1121 adjacent its proximal end. A manually graspable driver 1562 is attached to the proximal end of the lead 1164.

The tissue anchor 1550 of Figures 52 and 53 also includes an instrument marker 1130. The instrument marker 1130 may be carried by the lead 1560 adjacent a distal end thereof, *e.g.*, immediately adjacent the anchor members 1566. The instrument marker 1130 may be physically connected to a position detection system via wires or the like. Using wireless markers such as those described above in connection with Figures 2-8 and 23-33 as the marker 1130, though, permits the instrument marker 1130 and a wireless implanted marker to respond to the externally applied excitation energy from the same position detection system 1200 (Figure 21).

Figure 53 illustrates the tissue anchor 1550 with the anchor members 1566 in a deployment position. In Figure 53, the driver 1562 is retracted proximally. A majority, if not the entire length, of the anchor members 1566 may be received within the cannula 1552 of the introducer. The sharpened distal tip 1565 of the lead 1560 may be positioned immediately adjacent the distal end of the cannula 1552 to facilitate passage of the tissue anchor 1550 through the tissue to a desired location. Once that desired location is reached, as determined by comparing the position of the instrument marker 1130 with respect to a reference location, the driver 1562 may be manually advanced toward the position shown in Figure 52. This will move the anchor members 1566 of the tissue fastener from the stored position shown in Figure 52 to an anchoring position such as that shown in Figure 52 wherein the anchor members grasp the patient's tissue.

Figure 54 illustrates an alternative tissue anchor 1550a hot in accordance with the present invention. This tissue anchor 1550a is similar to the tissue anchor 1550 shown in Figures 52 and 53. However, in this example, the cannula 1552 of the introducer has a sharp distal end 1156 and a plurality of apertures (not seen in Figure 54, but analogous to apertures 330 in Figures 10 and 11) through which the anchor members 1566 may pass. As with the anchor 300 of Figures 9-11, a majority of the length of the anchor members 1566 of the tissue anchor 1550a may be retracted within the introducer during deployment of the tissue anchor to a desired location within the patient's tissue. Once that desired location has been reached, the tissue fastener may grasp tissue by advancing the anchor members 1566 through the apertures using the driver 1562. The driver 1562 may be manually actuated or the driver may include a motor 1168, *e.g.*, a precision stepper motor. This motor can be computer controlled to facilitate placement of the tissue anchor 1550a robotically, for example.

The embodiment of Figures 52 and 53 include a single instrument marker 1130 carried by the lead 1560 of the tissue anchor 1550. In the embodiment of Figure 54, the lead does not include an instrument marker. Instead, a first instrument marker 1130a and a second instrument marker 1130b are carried in the handle 1121. These two instrument markers may be positioned along an alignment axis. This alignment axis may be generally aligned with, or at least generally parallel to, the axis of the cannula 1552. As noted above in connection with the embodiment of Figure 41, this permits the position detection system 1200 (Figure 21) to determine the angle of the tissue anchor 1550a relative to a reference plane to facilitate display of the instrument on the user interface 1300.

Figure 55 shows another tissue anchor 1550b in accordance with a different embodiment of the invention. The handle 1121 of this embodiment includes a first instrument marker 1130a, a second instrument marker 1130b and a third instrument marker 1130c. The first instrument marker 1130a may be positioned along or adjacent to an axis of the tissue anchor 1550b, but the second and third instrument markers 1130c and 1130b are spaced away from the axis of the tissue anchor. Providing three spaced-apart markers in this fashion provides three position points for tracking by the position detection system 1200. As a result, the position detection system 1200 can determine both the angle and the rotational orientation of the tissue fastener 1550b with respect to a reference plane, as noted above in connection with Figure 42.

Figures 56-58 illustrate several additional embodiments of tissue anchors in accordance with the invention. In each of these embodiments, the tissue fastener includes a first set of proximally projecting anchor members 1566 and a second set of distally projecting anchor members 1567. In the illustrated embodiment, the proximally projecting anchor members are positioned proximally of the distally projecting anchor members such that they extend away from one another. These positions could be reversed, though, such that the anchor members extend toward one another when extended. Both sets of anchor members may be coupled to the same lead and moved from a deployment position within the cannula 1552 of the introducer to the tissue anchoring position shown in the drawings by moving the driver 1562 distally with respect to the introducer. While a tissue anchor of the invention may include any number of anchor members, using two opposed sets of anchor members extending in opposite directions, as shown, is believed to further stabilize the tissue grasped by the anchor members.

The tissue anchor 1550c of Figure 56 employs a single instrument marker 1130 carried adjacent a distal end of the cannula 1552 of the introducer. This will help pinpoint the position of the cannula's distal end with respect to an external reference location. However, it may not enable the position detection system 1200 to determine the orientation of the tissue anchor 1550c or the status of the tissue fastener, *e.g.*, whether it is in its stored position (with anchor members retracted) or anchoring position (with anchor members extended).

Figure 57 shows an alternative tissue anchor 1550d with the handle 1121 shown in partial cross-section. This embodiment includes a first instrument marker 1130a carried by a distal portion of the cannula 1552 and a second instrument marker 1130b carried by the lead 160 at a location spaced proximally of the first instrument marker, e.g., within the handle 1121. The first marker 1130a generates a first wireless signal in response to excitation by the position detection system and the second marker 1130b may generate a different second wireless signal in response to the same excitation. Providing one marker on the introducer and one on the lead will allow the position detection system to determine the angular orientation of the tissue anchor 1550d relative to a reference plane. In addition, the position detection system can determine the position of the second marker 1130b relative to the first marker 1130a and the relative distance between these markers. This enables the user to determine whether the anchor members 1566 and 1567 are in their deployment position within the cannula 1552 or, if not, how far they have been moved toward their fully extended position. The cannula 1552 may also include a series of graduations along some or all of its length. These graduations may be spaced from one another a standard distance, *e.g.*, 1 cm, giving the operator a direct measure of how far the tissue anchor has been advanced into the patient's tissue.

Figure 58 shows a further modification of this embodiment. The tissue anchor 1550e of Figure 58 is similar to the tissue anchor 1550d of Figure 57, but further includes a third instrument marker 1130c attached to the handle 1121. This third marker 1130c is spaced from an alignment axis extending between the first two markers, permitting the position detection system to further determine the rotational orientation of the tissue anchor 1550e, as noted above in connection with the embodiments of Figures 42 and 55.

Figures 59A-59D show the tissue anchor 1550d of Figure 57 with the anchor members 1566 and 1567 in various degrees of extension. In Figure 59A, the anchor members are received entirely within the cannula 1552. The driver 1562 is provided with two spaced-apart graduating rings 1569, visually dividing the driver into three segments. In Figure 59A, all three segments extend proximally beyond the handle 1121. In Figure 59B, the driver 1562 has been advanced one segment so that only one of the graduating rings 1569 is exposed. This advances the lead 1560 (Figure 57), thereby urging the anchor members 1566 and 1567 out of the cannula through apertures therein (not shown). In Figure 59C, the driver 1562 has been advanced one more segment, urging the anchor members 1566 and 1567 further out of the cannula and in Figure 59D the driver 1562 has been fully advanced, extending the anchor members 1566 and 1567 to their fullest extent. Figures 59A-59D illustrate that the degree of penetration of the anchor members into the tissue can be selectively controlled to yield a desired distance of penetration and extent of stabilization. The graduation rings 1569 give the user visible indicia on the tissue anchor 1550d itself which correlate to anchor member's penetration into the tissue. These indicia may be used in addition to position information displayed by the user interface based on the relative positions of the first and second markers 1130a and 1130b or could be used where the first and second markers are both carried on the lead or on the cannula and their distance would not change as the lead is advanced distally within the cannula.

Figure 60 illustrates a radio frequency (RF) ablation device 1170 having a function site which comprises an RF ablator 1172. The RF ablator 1172 includes RF elements 1174 extending outwardly from a central shaft 1175. These RF elements 1174 serves as RF electrodes, delivering RF energy to the tissue. The RF elements 1174 may be retractable into a cannula of an introducer in a manner similar to the tissue anchor 310 of Figures 9-11 or the tissue anchors 1150-1150e of Figures 52-58. The illustrated example includes ten RF elements 1174, but the number of RF elements can be varied. The ablator 1172 may also include a central cutting tip 1176 which serves as another RF cutting electrode. As with the other instruments 1120 discussed above, the instrument marker 1130 can be used to facilitate positioning of the ablator 1172, whereupon tissue surrounding the ablator may be ablated by application of RF energy to the tissue through the RF elements 1174.

Figure 61 illustrates a tissue anchor 1172a with four anchor members 1174a extending distally from the shaft 1175 and terminating in sharpened distal tips. These sharpened tips are arrayed about a sharpened central cutting tip 1176a. The sharpened tips of the anchor members 1174a and the sharp central tip 1176a facilitate passage of the tissue anchor through tissue during deployment in the patient's body. The tissue anchor 1172a may be used without RF energy, using sharp tips to advance the deployed device to the desired location. If this tissue anchor 1172a were used with RF energy, it is envisioned that the RF energy would be applied at relatively low levels or for relatively short durations to help advance the device through the tissue without ablatively destroying a large volume of tissue. Once the tissue anchor was in the desired location (which may be confirmed via the instrument marker 1130), RF energy delivery may be terminated and the anchor members could grasp the surrounding tissue, helping stabilize that tissue, as noted above. If this device were used in connection with softer tissue, *e.g.*, breast tissue, the tissue could collapse around the anchor members 1174a, further seating the tissue anchor 1172a in place.

Figures 62 and 63 illustrate RF activated tissue anchors in accordance with alternative embodiments of the invention. The RF activated tissue anchor 1172b of Figure 62 has four blunt-tipped anchor members 1174b arrayed around a blunt central cutting tip 1176b. The four anchor members and the central cutting tip may all serve as RF elements, delivering RF energy to the tissue to help advance the device through the tissue to the desired location. Once in place, RF energy may be stopped and the anchor members 1174b would grasp, and help stabilize, the surrounding tissue. In Figure 63, each of the anchor members 1174c of the tissue anchor 1172c has an insulative sheath along a portion of its length and an exposed length of metal at its distal tip. Similarly, the central cutting tip 1176c is insulated along a portion of its length, but the metal along its distal length is exposed. This helps localize the RF energy at the tips of the anchor members 1174c and the cutting tip 1176c to ease advancement of the tissue anchor 1170c through the tissue while minimizing collateral damage to surrounding tissue. As with the embodiments of Figures 61 and 62, by terminating application of RF energy to the tissue anchor 1170c the anchor members 1174c can grasp and help stabilize the tissue in which they reside.

Figure 73 illustrates a tissue anchor 1650 in accordance with an alternative embodiment of the invention. The tissue anchors 300 and 1550 discussed above rely on penetration of anchor members into the tissue to grasp and stabilize that tissue. The tissue anchor 1650 of Figure 73, though, utilizes a vacuum to grasp the tissue for stabilization.

The tissue anchor 1650 includes a hollow cannula 1660 having a plurality of perforations 1664 along a tissue fastening segment 1662. The cannula may be provided with a sharp distal tip 1665 to facilitate passage through tissue during placement of the device in the patient's tissue. One or more instrument markers 1130 (only two being shown in Figure 73) may be carried by the cannula 1660 1121 of the device to facilitate placement, as detailed above.

The tissue anchor 1650 is adapted for connection to a vacuum line 1668 which will reduce the pressure in the interior of the cannula. The vacuum line is operatively connected to a vacuum source 1670 and vacuum in the line 1668 can be activated or terminated, e.g., by means of a valve 1672. In initial deployment of the cannula to the desired location in the patient's tissue, the valve 1672 would be closed such that the interior of the cannula would not be at a reduced pressure, *e.g.*, it may be at ambient atmospheric pressure. Upon reaching the desired location, the valve 1672 can be opened, drawing a vacuum on the interior lumen of the cannula 1660. This lumen communicates with the surrounding tissue through the apertures 1664, sucking the tissue slightly into the apertures 1664 and against the exterior surface of the cannula 1660, thereby grasping the tissue. The apertures 1664 should be large enough and there should be enough apertures to adequately grasp the tissue, but the apertures should not be made so large as to aspirate the tissue into the cannula and break it away under the force of the vacuum.

The tissue fastening segment 1662 can be a fixed length, with the length selected to provide suitable fixation force for the anticipated range of uses. The tissue anchor 1650 of Figure 73, however, includes an axially slidable sleeve 1680 which can be used to control the effective length of the tissue fastening segment 1662. The sleeve is slidable along a length of the cannula so that is may selectively cover or expose some or all of the apertures 1664. The sleeve 1668 desirably closely engages the surface of the cannula to better seal apertures which it covers. Sliding the sleeve 1668 distally will reduce the effective exposed length of the tissue fastening segment 1662, while sliding the sleeve proximally will increase the effective length of the tissue fastening segment.

Figure 74 illustrates another instrument 1680. This instrument comprises a vacuum cutting device 1680 which is adapted to aspirate tissue from the patient's body adjacent the target site T. This vacuum cutting device has at least one distal aperture, typified in Figure 74 as an open distal tip 1684, which can be placed within the tissue adjacent the target site. The vacuum cutting device is operatively connected to a vacuum source 1670 via a vacuum line 1678. The vacuum within the interior of the vacuum cutting device 1680 can be controlled by, *e.g.*, a valve 1674. Tissue may be aspirated through the vacuum line 1678 to a container 1676 in which it may be collected for later analysis or disposal.

This vacuum cutting device 1680 may be used in conjunction with a tissue anchor, *e.g.*, any of the tissue anchors 300, 1550 and 1650 discussed above. If it is used in conjunction with the tissue anchor 1650 of Figure 73, as schematically suggested in Figure 74, the same vacuum source 1670 can be used with both the tissue anchor 1650 and the vacuum cutting device 1680. The vacuum cutting device 160 may sealingly engage the cannula 1660 of the tissue anchor, *e.g.*, via an o-ring 1682, to maintain a suitable vacuum within the device 1680. As with the cutter 200 and anchor 300 of Figures 18-20, the vacuum cutting device 1680 may be guided downwardly into the tissue along the cannula 1660 of the tissue anchor 1650. Grasping the tissue with the anchor 1650 will also stabilize a predetermined volume of tissue to be excised (highlighted in dashed lines in Figure 74). The anchor 1650 may be pulled upwardly during distal advancement of the vacuum cutting device 1680 to further stabilize the tissue.

The tissue anchor 1650 may be provided with at least one instrument marker 1130a and an instrument marker 1130b may be carried by the vacuum cutting device 1680. The relative positions of these instrument markers 1130a and 1130b and the implanted wireless marker 1100 can be used as a guide in excising the desired tissue.

One additional instrument (not illustrated) which can benefit from the application of instrument markers 1130 is a cryogenic probe. In accordance with this embodiment, a cryogenic probe is used to anchor tissue. The probe can be used to freeze a volume of tissue which encompasses all or a portion of the tissue which is to be removed. The cryogenic probe may serve as a handle for stabilizing and manipulating the target tissue. A marker can be carried by the probe to facilitate accurate placement of the cryogenic tip adjacent to a target location, which may be marked with a previously implanted marker. More than one marker may be placed in the shaft or handle of the cryogenic probe to aid in alignment of the cryogenic probe during correct placement in the tissue.

It will be appreciated that Figures 46-63, 73 and 74 illustrate only a few of the types of instruments for use with the system 1000 (Figure 21), and that other types of instruments can be used with the system 1000 by adding instrument markers 1130 that the position detection system 1200 can track.

D. User Interfaces

Figures 64-72 illustrate several examples of user interfaces 1300 and methods for using the systems 20 and 1000. The user interfaces 1300 can be used with any of the implantable markers 30 and 1100, and any of the instruments 200, 300 and 1120 described above with reference to Figures 1-63. The user interface 1300 is generally a computer display for graphically illustrating or otherwise presenting the position data generated by the position detection system 1200 to a user. The user interface 1300 can alternatively be an audio signal, a visual pattern based on light and/or color, a tactile or mechanical signal (*e.g.*, vibrational), or other indicators that can inform a physician of the relative position between the instrument and the target location.

Figure 64 is a schematic diagram illustrating an example of the system 1000 for displaying the relative position between an instrument 1120 and the target location T. In this example, the system 1000 includes an implantable marker 1100 implanted in the body part B, an instrument 1120 for performing a procedure on the target location T, the position detection system 1200, and the user interface 1300. The implantable marker 1100 and the instrument 1120 can be any one of the examples of these devices described above. The instrument 1120, more specifically, has an instrument coordinate system 1129 defined by the orthogonal axes $X_i$-$Y_i$-$Z_i$. The $Z_i$-axis is aligned with the alignment axis A-A, and the $X_i$-axis and $Y_i$-axis define an operating plane normal to the $Z_i$-axis. The instrument coordinate system 1129 moves with the instrument during the procedure. The position detection system 1200 generally includes the same components described above with reference to Figures 21 and 22. As such, the position detection system 1200 can include an array 1204 having sensors 1210 and a transmitter 1220 for emitting an excitation energy that drives the implanted marker 1100 and the instrument markers 1130. The position detection system 1200 can also include a reference coordinate system 1212 defined by three orthogonal axes $X_r$-$Y_r$-$Z_r$. In operation, the position detection system 1200 determines the position of the implanted marker 1100 and the positions of the instrument markers 1130 relative to the reference coordinate system 1212 to determine the relative position between the function-site 1124 of the instrument 1120 and the target location T. The position detection system 1200 can also include a processor.

The user interface 1300 provides a display or another type of indicator of the relative position between the function-site 1124 and the target location T based on data from the position detection system 1200. In this example, the user interface 1300 includes a processor 1302, a memory 1304 coupled to the processor 1302, an input device 1306 for controlling parameters of the system 1000, and an output display 1310. The processor 1302 and the memory 1304 can be a computer available from many sources. The input device 1306 can be a keyboard, a computer mouse, a touch screen, or any other suitable device for inputting commands to the processor 1302. The output display 1310 is preferably a display screen, but it can also be another type of output device that generates an output that can be detected and understood by a user. The user interface 1300 also includes a display coordinate system 1308 defined by three orthogonal axes $X_d$-$Y_d$-$Z_d$. The display coordinate system 1308 can initially correspond to the reference coordinate system 1212 of the position detection system 1200. In many applications, however, it may not be desirable to view the display 1310 based upon the reference coordinate system 1212. The processor 1302 can accordingly calibrate the display coordinate system 1308 so that the display 1310 shows a desired two-dimensional plane or a desired three-dimensional space.

In operation, the user interface 1300 processes data from the position detection system 1200 in real-time to show the relative motion between the function-site 1124 and the target location T. For example, the processor 1302 receives signals from the position detection system 1200 and produces output signals that can be represented by the output display 1310. As explained in more detail below, the user can set the parameters for generating the virtual margin 1301 and controlling other aspects of the user interface 1300 using the input device 1306.

Figure 64 also illustrates an orientation between the instrument 1120 and the target location T that generally corresponds to a calibrating stage of a procedure for treating, probing, or monitoring the target location T. The surgeon typically holds the instrument 1120 so that the alignment axis A-A of the instrument 1120 defines a desired Zi elevation axis along which the surgeon moves the instrument 1120 up and down relative to the target location T. The Xi-$Y_i$ plane normal to the $Z_i$-axis defines the desired operating plane in which the surgeon moves the instrument 1120 along a margin M around the target location T during a procedure. When the physician holds the instrument 1120 relative to the target location T in a desired orientation for performing the procedure, the instrument coordinate system 1129 ($X_i$-$Y_i$-$Z_i$) may not be aligned with the reference coordinate system 1212 ($X_r$-$Y_r$-$Z_r$) and the display coordinate system 1308 ($X_d$-$Y_d$-$Z_d$). The user interface 1300 accordingly calibrates the display coordinate system 1308 to coincide with the instrument coordinate system 1129 so that the user interface 1300 indicates movement of instrument 1120 (a) along the alignment axis A-A as an elevation relative to the target location T, and (b) through the operating plane $X_i$-$Y_i$ as a location in an X-Y grid of the display 1310.

Figure 65 illustrates one example of the user interface 1300 showing the relative position between the instrument 1120 and the target location T before calibrating the position detection system 1200 to align the display coordinate system 1308 (Figure 64) with the instrument coordinate system 1129 (Figure 64). In this example, the display 1310 has a two-dimensional grid 1320 that shows the $X_d$-$Y_d$ plane of the display coordinate system 1308. The display 1310 can also include a numerical elevation indicator 1332 and/or a graphical elevation indicator 1334. The elevation indicators 1132 and 1134 show the position along the $Z_d$-axis of the display coordinate system 1308. The instrument 1120 is displayed as a line on the grid 1320 because the user interface 1300 has not yet been calibrated to align the display coordinate system 1308 with the instrument coordinate system 1129. The function-site 1124 of the instrument 1120 appears as a point at one end of instrument 1120, and the elevation of the function-site 1124 relative to the target location T is displayed by one or both of the elevation indicators 1332 and 1334. At this stage before calibrating the user interface 1300, it may be difficult for a physician to determine the relative position between the function-site 1124 and the target location T because moving the instrument 1120 along the alignment axis A-A simultaneously changes the position of the function-site 1124 on the grid 1320 and on the elevation indicators 1332 and 1334. Therefore, to provide a more intuitive display of the motion of the instrument 1120, the position detection system 1200 aligns the display coordinate system 1308 with the instrument coordinate system 1129.

Referring to Figure 54B, an example of an algorithm for performing the calibration transformation is described as follows. The definitions include Azimuth = $\psi$; Elevation = $\theta$; Point before transformation = (*a,b,c*). The mathematical equation to convert this point into the X',Y',Z' coordinate system; (a',b',c'). In the user interface, the marker would be at (0,0,0) after the implementation of the algorithms. The X, Y, Z axis would still be oriented with the original coordinate system of the system reference. First rotate about the z-axis by the azimuth angle or $\psi$. The point in this intermediary coordinate system is now defined as:

$$p = a*\cos(\psi) + b*\sin(\psi)$$

$$q = b*\cos(\psi) - a*\sin(\psi)$$

$$r = c$$

Next, rotate about the y-axis so that the z-axis is in line with the probe. Effectively rotation will be about the y-axis by the elevation angle - 90° or ($\theta$ - 90°). The point in the X',Y',Z' coordinate system would now be defined as:

$$a' = p*\sin(\theta) - r*\cos(\theta)$$

$$b' = q$$

$$c' = p*\cos(\theta) + r*\sin(\theta)$$

Substituting the values of p, q, and r into these equations the following equation is obtained in terms of the original coordinates and the azimuth and elevation angles:

$$a' = [a*\cos(\psi) + b*\sin(\psi)]* \sin(\theta) - c*\cos(\theta)$$

$$b' = b*\cos(\psi) - a*\sin(\psi)$$

$$c' = [a*\cos(\psi) + b*\sin(\psi)]*\cos(\theta) + c*\sin(\theta)$$

The point (a',b',c') represents the original point (a,b,c) transformed into the new coordinate system. The user interface display probe tip projection math length projection on X-Y display plane is defined by the equation:

## Display Length = length probe tip * cosine (Elevation angle)

Based on these algorithms, a person skilled in the art can program the user interface 1300 to perform the calibration without undue experimentation.

Figure 66 illustrates an example of the user interface 1300 of Figure 65 after the position detection system 1200 calibrates the user interface 1300 to align the display coordinate system 1308 with the instrument coordinate system 1129. In this example, the instrument 1120 and the function-site 1124 are both displayed as a point location on the grid 1320. The elevation of the function-site 1124 relative to the target location T still appears as a numeric or graphical readout on the elevation indicators 1332 and 1334. After calibrating the coordinate systems, the user interface 1300 accordingly shows (a) movement of the instrument 1120 solely along the alignment axis A-A by changing only the readout on the elevation indicators 1332 and 1334 without changing the location of the instrument 1120 on the grid 1320, and (b) movement of the instrument 1120 solely through the operating plane $X_i$-$Y_i$ by changing only the location of the instrument 1120 on the grid 1320 without changing the readout on the elevation indicators 1332 and 1334. The position detection system 1200 and/or the user interface 1300 can alternatively continuously calibrate the system 1000 so that the display coordinate system 1308 continuously coincides with the instrument coordinate system 1129. In such an example, the grid 1320 is continuously normal to the alignment axis A-A of the instrument 1120 such that the display 1310 continuously displays the instrument 1120 as a point location (as shown in Figure 55) irrespective of the orientation of the instrument 1120.

Figures 65 and 66 also illustrate one example for defining a virtual margin 1301 relative to the target location T for use on the display 1310 of the user interface 1300. As described above with reference to Figures 34-39, the virtual margin 1301 can be generated based upon the position of an implantable marker 1100 or a plurality of implantable markers 1100. The virtual margin 1301 is generally defined by a physician based upon information from an imaging procedure, such as when the markers 1100 are implanted. The virtual margin 1301 can be configured to include a lesion, tumor, or other mass that defines the area of interest at the target location T. The virtual margin 1301 should be configured to avoid removing or otherwise performing a procedure on material outside of the virtual margin 1301. As such, after determining the relative position between the implantable marker 1100 and the target location T using an imaging process (*e.g.*, radiation, MRI, ultrasound, etc.), the physician determines the desired virtual margin 1301 to input into the user interface 1300.

The physician can input the desired virtual margin 1301 into the user interface 1300 using the input device 1306 of the user interface 1300 or an instrument 1120 (*e.g.*, the probe 1120f shown in Figure 51). In one example using a keyboard, the physician can enter a desired radius relative to the target location T to define a spherical or cylindrical virtual margin 1301 that is displayed as a circle on the grid 1320 of the display 1310. As explained above, the virtual margin 1301 can also be configured to be rectilinear, a compound shape, or any other suitable two-dimensional or three-dimensional shape that is defined by the physician. The user interface 1300 accordingly displays the selected a virtual margin 1301 to define a boundary relative to the target location T. For example, the virtual margin 1310 is often configured to completely surround or encompass a tissue mass or other body part within the target location T. Referring still to Figures 65 and 66, this example of the invention illustrates a single implantable marker 1100 disposed in the target location T and a spherical or cylindrical virtual margin 1301 around the implantable marker 1100.

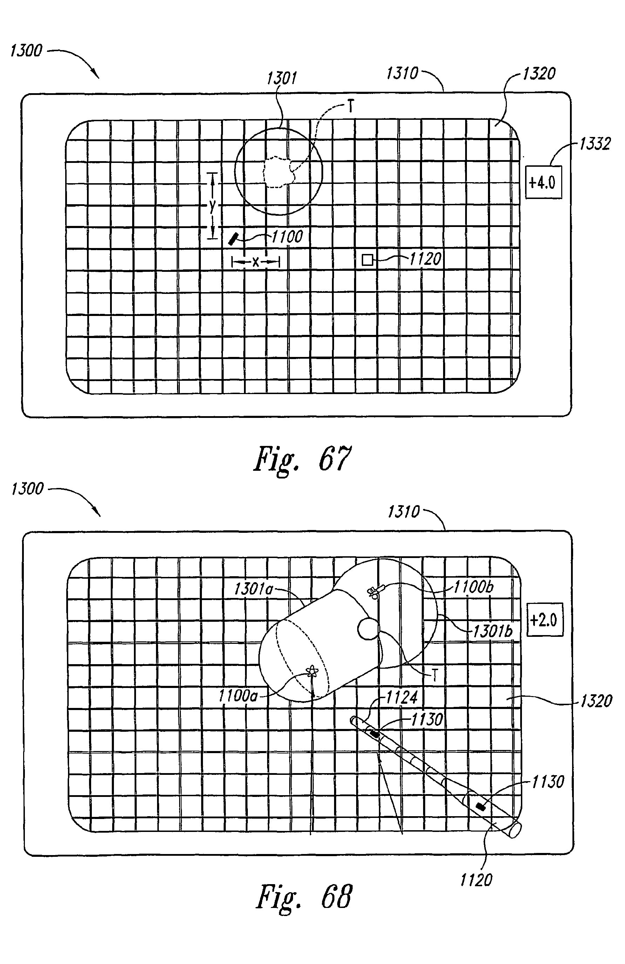

Figure 67 illustrates another example for defining a virtual margin 1301 relative to a target location T. In this example, the user interface 1300 can display an outline of the target location T (shown in broken lines), but it will be appreciated that the target location T may not be displayed on the grid 1320. This example illustrates a single implantable marker 1100 disposed outside of the target location T by an offset distance having coordinate differentials of "X" along an X-axis of the grid 1320, "Y" along the Y-axis of the grid 1320, and "Z" (not shown) along an axis normal to a plane defined by the grid 1320. The offset distance can be determined during a previous imaging procedure or when the implantable marker 1100 is implanted using known radiation, MRI, ultrasound and other imaging techniques. Based upon the position of the implantable marker 1100 and the offset distance between the implantable marker 1100 and the target location T, the user interface 1300 can generate the virtual margin 1301 around the actual location of the target location T. One advantage of implanting the marker 1100 outside of the target location T is that the implantable marker 1100 does not pierce the tissue mass or other body part of the target location T. This feature can be particularly useful in applications for removing cancerous tissue masses or other types of tissue/bone masses that are desirably left intact until they are removed from the patient.

Figure 68 illustrates another example for defining a virtual margin 1301 relative to the target location T. In this example, two implantable markers 1100a and 1100b have been implanted at two separate offset distances relative to the target location T. The physician can input two separate virtual margins 1301 a and 1301 b relative to the individual implantable markers 11100a and 1100b, respectively. In this particular example, the virtual margin 1301 a is relative to the first implantable marker 1100a and defines a cylindrical boundary. Similarly, the virtual margin 1301b is relative to the second implantable marker 1100b, but it defines a spherical boundary. The virtual margins 1301 a and 1301 b together define a compound virtual margin relative to the target location T. It will be appreciated that several other virtual margins can be developed using different combinations of one or more implantable markers, and different combinations of markers that are implanted in and/or offset from the target location T. In any of the examples of the virtual margins 1301 described above, the physician can manipulate an instrument 1120 relative to the target location T using the user interface 1300 to display the relative position between the function-site 1124 of the instrument 1120 relative to the virtual margin 1301.

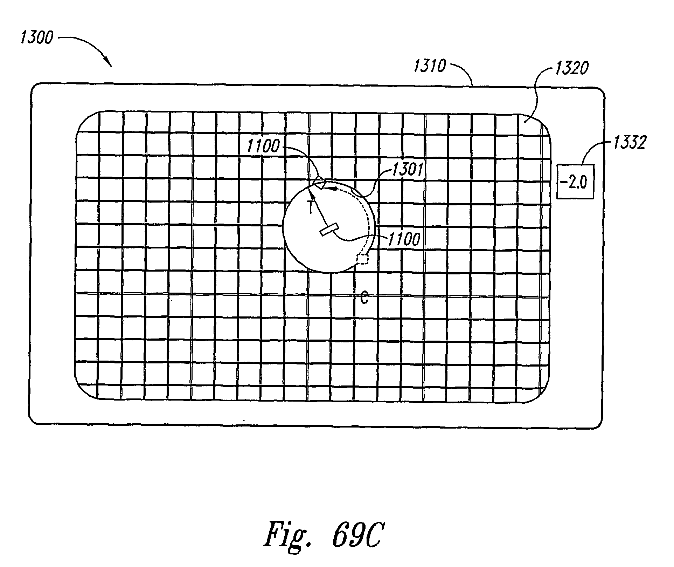

Figures 69A-69C illustrate a procedure for operating the system 1000. In this example, a single implantable marker 1100 has been implanted within the target location T and the user interface 1300 has generated a cylindrical or spherical virtual margin 1301 around the target location T. Referring to Figure 69C, the instrument 1120 is shown after the display coordinate system has been calibrated to be aligned with the instrument coordinate system in the manner explained above with reference to Figures 53-55. The user interface 1300 initially displays the instrument 1120 as a point at a location A. Based upon this display, the physician understands that the alignment axis A-A of the instrument 1120 is normal to the grid 1320 of the display 1310, and that the function-site 1124 of the instrument 1120 is at an elevation of 5 cm above a predetermined reference plane relative to the target location T and/or the implanted marker 1100 (see the elevation indicator 1332). The physician then moves the instrument 1120 transverse relative to the alignment axis A-A to a location B on the virtual margin 1301. In this particular example, the physician held the instrument 1120 at a constant elevation of 5 cm above the reference plane shown by the elevation indicator 1132.

Figure 69 illustrates a subsequent stage of operating the system 1000. After moving the instrument 1120 from location A to location B (Figure 69A), the physician inserts the function-site 1124 of instrument 1120 into the body part to move the function-site 1124 from the location B to a location C. Referring to both Figures 69A and 69B, the elevation indicator 1132 shows that the elevation of the function-site 1124 relative to the reference plane has moved from 5 cm above the reference plane to 2 cm below the reference plane. In an application in which the physician wants to excise a cylindrical tissue mass having a base 2 cm below the reference plane, the instrument 1120 at location C is accordingly ready to be moved along the virtual margin 1301 to excise a mass of tissue. Referring to Figure 69, the user interface 1300 displays the motion of the instrument as the physician or robot moves it along the virtual margin 1301. The virtual margin 1301 accordingly provides a guide to the physician that allows the physician to excise a precise volume of tissue without cutting into the target mass or damaging tissue outside of the target location T.

Figure 70 illustrates another example of the user interface 1300. In this example, the display 1310 includes a first grid 1320 illustrating a top view relative to a reference plane and a second grid 1420 illustrating a front view normal to the reference plane. For purposes of convention, the reference plane can be parallel to the table on which the patient is positioned during a procedure, but it can also be at an angle to the table. The display 1310 can also include an elevation indicator 1132 showing the elevation of the function-site 1124 relative to the target location T and a distance indicator 1432 showing the point-to-point distance between the function-site 1124 and the target location T. The example of the display 1310 shown in Figure 70 provides the physician two separate views that the physician can use to more accurately position the function-site 1124 relative to the target location T. The operation and the advantages of the display 1310 illustrated in Figure 70 are expected to be similar to those described above with reference to Figures 66-68.

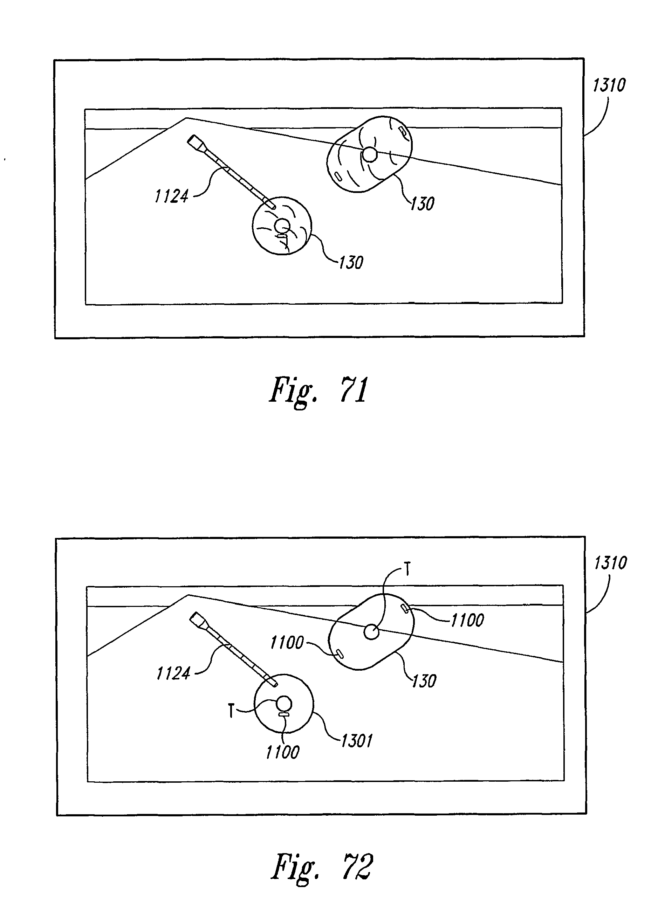

Figures 71 and 72 illustrate additional examples of the user interface 1300. Referring to Figure 71, the display 1310 provides a three-dimensional solid or opaque representation of the virtual margin 1301. Figure 72 illustrates an embodiment in which the display 1310 provides a holographic representation of the virtual margin 1301 such that the target location T can be represented within the holographic representation. Suitable software for generating the three-dimensional representations of the virtual margin 1301 illustrated in Figures 71 and 72 is available from Medical Media System of West Lebanon, New Hampshire. The three-dimensional representations of the virtual margin 1301 also provide a physician with an - intuitive understanding of the relative position between the function-site 1124 of the instrument 1120 and the virtual margin 1301 relative to the target location T. It is expected, therefore, that the three-dimensional virtual margins 1301 will also allow physicians to accurately perform procedures or monitor internal target locations within a human body without additional imaging equipment or procedures.

**Claims**

**1.** A tissue anchor for holding a tissue mass within a human body, comprising:

a lead (304, 1560);
a tissue fastener (1160) coupled to the lead (304); and
a first implantable wireless marker (30, 1130) carried by the lead (304, 1560) and/or the tissue fastener (1160), wherein the first wireless marker (30, 1130) generating, in response to an externally applied excitation energy, a first wireless signal which is adapted to be detected by a position detection system to determine a position of the first wireless marker (30, 1130) relative to a reference location,

wherein the first wireless marker (30, 1130) is capable of emitting a first radio frequency in response to a magnetic field, ultrasound or a second, different, radio frequency

2. A tissue anchor of claim 1 for use in connection with a wireless implantable marker (1100) implanted in a human body, wherein the implantable marker (1100) generates a wireless signal that can be detected by a position detection system external to the human body in response to an excitation energy applied by the position detection system,

3. The tissue anchor of claim 1 further comprising a second wireless marker (30, 1130) carried by the lead (304, 1560) and/or the tissue fastener (1160) at a location spaced from the first wireless marker (30, 1130), the second wireless marker (30,1130) generating, in response to the externally applied excitation energy, a second wireless signal that can be detected by the position detection system to determine a position of the second wireless marker (30, 1130) relative to the position of the first wireless marker (30, 1130).

4. The tissue anchor of claim 3 wherein the first wireless signal is different from the second wireless signal, permitting the position detection system to differentiate between the first wireless marker (30, 1130a) and the second wireless marker (30,1130b)

5. The tissue anchor of claim 3 wherein the first and second wireless markers (30, 1130) are spaced from one another along an alignment axis to permit the position detection system to determine angular orientation of the tissue anchor with respect to a reference plane.

6. The tissue anchor of claim 5 further comprising a third wireless marker (30, 1130c) carried by the lead (304, 1560) and/or the tissue fastener (1160) at a location spaced from the alignment axis, the third wireless marker (30, 1130c) generating, in response to the externally applied excitation energy, a third wireless signal that can be detected by the position detection system to determine rotational position of the anchor about the alignment axis.

7. The tissue anchor of any preceding claim further comprising an introducer (320, 1552) which maintains the tissue fastener (1160) in a deployment position for deployment in tissue, but permits the tissue fastener (1160) to move to an anchoring position wherein it may grasp tissue,

8. The tissue anchor of claim 7 further comprising a wireless introducer marker (30, 1130) carried by the introducer (320, 1552), the wireless introducer marker generating, in response to the externally applied excitation energy, a wireless introducer signal that can be detected by the position detection system to determine a position of the wireless introducer marker relative to the position of the first wireless marker (30).

9. A tissue stabilizer system, comprising:

an introducer (320, 1552); and
an anchor (300, 1120, 1550, 1650, 1680) according to claim 1 carried by the introducer (320), and the tissue fastener (1160) being moveable from a stored position to an anchoring position wherein it may grasp tissue

10. The tissue stabilizer system of claim 9 wherein the introducer (320, 1552) comprises an elongate tube within which at least a portion of the lead (304, 1500) is received, the lead (304, 1560) being moveable with respect to the introducer (320) to urge the tissue fastener (1160) from the stored position to the anchoring position,

11. The tissue stabilizer system of claim 10 wherein the elongate tube includes at least one aperture (330), the tissue fastener (1160) being advanced through the aperture (330) in moving from the stored position to the anchoring position.

12. The tissue stabilizer system of Claims 9-11, wherein the introducer (320, 1552) has a sharpened distal tip (326) adapted to advance through the tissue to position the tissue fastener (1160) within the tissue at a desired location prior to movement of the tissue fastener (1160) from its stored position to its anchoring position

13. The tissue stabilizer system of Claims 9-12, further comprising a driver coupled to the lead (304, 1560), the driver urging the lead (304, 1560) with respect to the introducer (320) to move the tissue fastener (1160) from its deployment position to its anchoring position.

14. The tissue stabilizer system of claim 13 wherein the driver (1568) comprises a manually graspable handle (1121), which a user may manually move to so urge the lead (304, 1560), or a motor (1168).

15. The tissue stabilizer system of claim 14 wherein the driver (1568) comprises a motor (1168), further comprising a controller associated with the motor to control advancement of the introducer (320, 1552) in response to position information received from the position detection system.

16. The anchor or system of any preceding claim wherein the first wireless marker includes an electronic circuit resonator which generates the first wireless signal or a magnetic resonator which generates the first wireless signal or a radio frequency marker which generates the first wireless signal,

**Patentansprüche**

1. Gewebeanker zum Halten einer Gewebemasse in einem menschlichen Körper, umfassend:

ein Führungselement (304, 1560);
ein Gewebeverbindungselement (1160), das mit dem Führungselement (304) verbunden ist; und
einen ersten implantierbaren Drahtlos-Marker (30, 1130), der von dem Führungselement (304, 1560) und/oder dem Gewebeverbindungselement (1160) gehalten wird, wobei der erste Drahtlos-Marker (30, 1130) als Reaktion auf eine extern angelegte Anregungsenergie ein erstes Drahtlos-Signal erzeugt, das dazu eingerichtet ist, durch ein Positionserkennungssystem erkannt zu werden, um eine Position des ersten Drahtlos-Markers (30, 1130) in Bezug auf eine Referenzstelle zu ermitteln,

wobei der erste Drahtlos-Marker (30, 1130) in der Lage ist, eine erste Funkfrequenz als Reaktion auf ein Magnetfeld, auf Ultraschall oder auf eine zweite, unterschiedliche Funkfrequenz auszusenden.

2. Gewebeanker gemäß Anspruch 1 zur Verwendung in Verbindung mit einem implantierbaren Drahtlos-Marker (1100), der in einen menschlichen Körper implantiert ist, wobei der implantierbare Marker (1100) ein Drahtlos-Signal erzeugt, das durch ein sich außerhalb des menschlichen Körpers befindliches Positionserkennungssystem erkannt werden kann, und zwar als Reaktion auf eine durch das Positionserkennungssystem angelegte Anregungsenergie.

3. Gewebeanker gemäß Anspruch 1, des Weiteren umfassend einen zweiten Drahtlos-Marker (30, 1130), der von dem Führungselement (304, 1560) und/oder dem Gewebeverbindungselement (1160) an einer Stelle gehalten wird, die einen Abstand zum ersten Drahtlos-Marker (30, 1130) aufweist, wobei der zweite Drahtlos-Marker (30, 1130) als Reaktion auf die extern angelegte Anregungsenergie ein zweites Drahtlos-Signal erzeugt, das durch das Positionserkennungssystem erkannt werden kann, um eine Position des zweiten Drahtlos-Markers (30, 1130) in Bezug auf die Position des ersten Drahtlos-Markers (30, 1130) zu ermitteln.

4. Gewebeanker gemäß Anspruch 3, wobei sich das erste Drahtlos-Signal von dem zweiten Drahtlos-Signal unterscheidet, wodurch dem Positionserkennungssystem ermöglicht wird, zwischen dem ersten Drahtlos-Marker (30, 1130a) und dem zweiten Drahtlos-Marker (30, 1130b) zu unterscheiden.

5. Gewebeanker gemäß Anspruch 3, wobei die ersten und zweiten Drahtlos-Marker (30, 1130) mit einem Abstand zueinander entlang einer Ausrichtungsachse angeordnet sind, um dem Positionserkennungssystem zu ermöglichen, die Winkelorientierung des Gewebeankers in Bezug auf eine Referenzebene zu ermitteln.

6. Gewebeanker gemäß Anspruch 5, des Weiteren umfassend einen dritten Drahtlos-Marker (30, 1130c), der von dem Führungselement (304, 1560) und/oder dem Gewebeverbindungselement (1160) an einer Stelle gehalten wird, die einen Abstand zur Ausrichtungsachse aufweist, wobei der dritte Drahtlos-Marker (30,1130c) als Reaktion auf die extern angelegte Anregungsenergie ein drittes Drahtlos-Signal erzeugt, das durch das Positionserkennungssystem erkannt werden kann, um die Drehposition des Ankers um die Ausrichtungsachse herum zu ermitteln.

7. Gewebeanker gemäß einem der vorherigen Ansprüche, des Weiteren umfassend ein Einführelement (320, 1552), welches das Gewebeverbindungselement (1160) in einer Einsatzposition hält, um es in Gewebe einsetzen zu können, aber dem Gewebeverbindungselement (1160) ermöglicht, sich in eine Verankerungsposition zu bewegen, in der es in Gewebe eingreifen kann.

**8.** Gewebeanker gemäß Anspruch 7, des Weiteren umfassend einen durch das Einführelement (320, 1552) gehaltenen Drahtlos-Einführelement-Marker (30, 1130), wobei der Drahtlos-Einführelement-Marker als Reaktion auf die extern angelegte Anregungsenergie ein Drahtlos-Einführelement-Signal erzeugt, das durch das Positionserkennungssystem erkannt werden kann, um eine Position des Drahtlos-Einführelement-Markers in Bezug auf den ersten Drahtlos-Marker (30) zu ermitteln.

**9.** Gewebestabilisierungssystem, umfassend:

ein Einführelement (320, 1552); und
einen Anker (300, 1120, 1550, 1650, 1680) gemäß Anspruch 1, der durch das Einführelement (320) gehalten wird, wobei das Gewebeverbindungselement (1160) aus einer verstauten Position in eine Verankerungsposition bewegt werden kann, in der es in Gewebe eingreifen kann.

**10.** Gewebestabilisierungssystem gemäß Anspruch 9, wobei das Einführelement (320, 1552) ein langes und dünnes Rohr umfasst, in dem mindestens ein Abschnitt des Führungselements (304, 1500) aufgenommen wird, wobei das Führungselement (304, 1560) in Bezug auf das Einführelement (320) bewegt werden kann, um das Gewebeverbindungselement (1160) aus der verstauten Position in die Verankerungsposition zu drängen.

**11.** Gewebestabilisierungssystem gemäß Anspruch 10, wobei das lange und dünne Rohr mindestens eine Öffnung (330) enthält, wobei das Gewebeverbindungselement (1160) durch die Öffnung (330) ausgeschoben wird, wenn es aus der verstauten Position in die Verankerungsposition bewegt wird.

**12.** Gewebestabilisierungssystem gemäß den Ansprüchen 9-11, wobei das Einführelement (320, 1552) eine geschärfte distale Spitze (326) hat, die dazu angepasst ist, durch das Gewebe vorzudringen, um das Gewebeverbindungselement (1160) im Gewebe an einer gewünschten Stelle zu positionieren, bevor das Gewebeverbindungselement (1160) aus seiner verstauten Position in seine Verankerungsposition bewegt wird.

**13.** Gewebestabilisierungssystem gemäß den Ansprüchen 9-12, des Weiteren umfassend ein mit dem Führungselement (304, 1560) verbundenes Antriebselement, wobei das Antriebselement das Führungselement (304, 1560) in Bezug auf das Einführelement (320) drängt, das Gewebeverbindungselement (1160) aus seiner Einsatzposition in seine Verankerungsposition zu bewegen.

**14.** Gewebestabilisierungssystem gemäß Anspruch 13, wobei das Antriebselement (1568) einen manuell ergreifbaren Handgriff (1121), den ein Anwender manuell bewegen kann, um das Führungselement (304, 1560) zu drängen, oder einen Motor (1168) umfasst.

**15.** Gewebestabilisierungssystem gemäß Anspruch 14, wobei das Antriebselement (1568) einen Motor (1168) umfasst, des Weiteren ein zu dem Motor gehörendes Steuerelement zur Steuerung des Vordringens des Einführelements (320, 1552) als Reaktion auf Positionsinformationen, die von dem Positionserkennungssystem empfangen werden.

**16.** Anker oder System gemäß einem der vorherigen Ansprüche, wobei der erste Drahtlos-Marker einen Schaltkreis-Resonator enthält, der das erste Drahtlos-Signal erzeugt, oder einen Magnet-Resonator enthält, der das erste Drahtlos-Signal erzeugt, oder einen Funkfrequenz-Marker enthält, der das erste Drahtlos-Signal erzeugt.

## Revendications

**1.** Un ancrage tissulaire destiné à maintenir une masse tissulaire à l'intérieur d'un corps humain, comprenant :

un fil (304, 1560),
un dispositif de fixation tissulaire (1160) couplé au fil (304), et
un premier marqueur sans fil implantable (30, 1130) transporté par le fil (304, 1560) et/ou le dispositif de fixation tissulaire (1160), où le premier marqueur sans fil (30, 1130) génère, en réponse à une énergie d'excitation appliquée de manière externe, un premier signal sans fil qui est adapté de façon à être détecté par un système de détection de position de façon à déterminer une position du premier marqueur sans fil (30, 1130) par rapport à un emplacement de référence,

où le premier marqueur sans fil (30, 1130) est capable d'émettre une première fréquence radio en réponse à un champ magnétique, un ultrason ou une deuxième fréquence radio différente.

2. Un ancrage tissulaire selon la revendication 1 destiné à une utilisation en relation avec un marqueur sans fil implantable (1100) implanté dans un corps humain, où le marqueur implantable (1100) génère un signal sans fil qui peut être détecté par un système de détection de position externe au corps humain en réponse à une énergie d'excitation appliquée par le système de détection de position.

3. L'ancrage tissulaire selon la revendication 1 comprenant en outre un deuxième marqueur sans fil (30, 1130) transporté par le fil (304, 1560) et/ou le dispositif de fixation tissulaire (1160) à un emplacement espacé du premier marqueur sans fil (30, 1130), le deuxième marqueur sans fil (30, 1130) générant, en réponse à l'énergie d'excitation appliquée de manière externe, un deuxième signal sans fil qui peut être détecté par le système de détection de position de façon à déterminer une position du deuxième marqueur sans fil (30, 1130) par rapport à la position du premier marqueur sans fil (30, 1130).

4. L'ancrage tissulaire selon la revendication 3 où le premier signal sans fil est différent du deuxième signal sans fil, permettant au système de détection de position de faire une différenciation entre le premier marqueur sans fil (30, 1130a) et le deuxième marqueur sans fil (30, 1130b).

5. L'ancrage tissulaire selon la revendication 3 où les premier et deuxième marqueurs sans fil (30, 1130) sont espacés l'un de l'autre le long d'un axe d'alignement de façon à permettre au système de détection de position de déterminer une orientation angulaire de l'ancrage tissulaire par rapport à un plan de référence.

6. L'ancrage tissulaire selon la revendication 5 comprenant en outre un troisième marqueur sans fil (30, 1130c) transporté par le fil (304, 1560) et/ou le dispositif de fixation tissulaire (1160) à un emplacement espacé de l'axe d'alignement, le troisième marqueur sans fil (30, 1130c) générant, en réponse à l'énergie d'excitation appliquée de manière externe, un troisième signal sans fil qui peut être détecté par le système de détection de position de façon à déterminer une position de rotation de l'ancrage autour de l'axe d'alignement.

7. L'ancrage tissulaire selon l'une quelconque des revendications précédentes comprenant en outre un introducteur (320, 1552) qui maintient le dispositif de fixation tissulaire (1160) dans une position de déploiement pour un déploiement dans les tissus, mais qui permet au dispositif de fixation tissulaire (1160) de se déplacer vers une position d'ancrage où il peut saisir les tissus.

8. L'ancrage tissulaire selon la revendication 7 comprenant en outre un marqueur d'introducteur sans fil (30, 1130) transporté par l'introducteur (320, 1552), le marqueur d'introducteur sans fil générant, en réponse à l'énergie d'excitation appliquée de manière externe, un signal d'introducteur sans fil qui peut être détecté par le système de détection de position de façon à déterminer une position du marqueur d'introducteur sans fil par rapport à la position du premier marqueur sans fil (30).

9. Un système de stabilisation tissulaire, comprenant :

un introducteur (320, 1552), et
un ancrage (300, 1120, 1550, 1650, 1680) selon la revendication 1 transporté par l'introducteur (320), et le dispositif de fixation tissulaire (1160) étant déplaçable d'une position rangée vers une position d'ancrage où il peut saisir les tissus.

10. Le système de stabilisation tissulaire selon la revendication 9 où l'introducteur (320, 1552) comporte un tube allongé à l'intérieur duquel au moins une partie du fil (304, 1500) est reçue, le fil (304, 1560) étant déplaçable par rapport à l'introducteur (320) de façon à pousser le dispositif de fixation tissulaire (1160) de la position rangée vers la position d'ancrage.

11. Le système de stabilisation tissulaire selon la revendication 10 où le tube allongé est muni d'au moins une ouverture (330), le dispositif de fixation tissulaire (1160) étant avancé au travers de l'ouverture (330) lors du déplacement de la position rangée vers la position d'ancrage.

12. Le système de stabilisation tissulaire selon les revendications 9 à 11, où l'introducteur (320, 1552) possède une extrémité distale affûtée (326) adaptée de façon à avancer au travers des tissus de façon à positionner le dispositif de fixation tissulaire (1160) à l'intérieur des tissus à un emplacement souhaité avant le déplacement du dispositif de fixation tissulaire (1160) de sa position rangée vers sa position d'ancrage.

13. Le système de stabilisation tissulaire selon les revendications 9 à 12, comprenant en outre un système d'entraînement couplé au fil (304, 1560), le système d'entraînement poussant le fil (304, 1560) par rapport à l'introducteur (320) de façon à déplacer le dispositif de fixation tissulaire (1160) de sa position de déploiement vers sa position d'ancrage.

14. Le système de stabilisation tissulaire selon la revendication 13 où le système d'entraînement (1568) comporte une poignée à préhension manuelle (1121), qu'un utilisateur peut déplacer manuellement de façon à pousser le fil (304, 1560), ou un moteur (1168).

15. Le système de stabilisation tissulaire selon la revendication 14 où le système d'entraînement (1568) est muni d'un moteur (1168), comprenant en outre un système de commande associé au moteur de façon à commander l'avancée de l'introducteur (320, 1552) en réponse à des informations de position reçues du système de détection de position.

16. L'ancrage tissulaire ou le système selon l'une quelconque des revendications précédentes où le premier marqueur sans fil est muni d'un résonateur de circuit électronique qui génère le premier signal sans fil ou d'un résonateur magnétique qui génère le premier signal sans fil ou d'un marqueur de fréquence radio qui génère le premier signal sans fil.

Fig. 1

Fig. 1A

Fig. 1B

30a — 40

Fig. 2A

42

30b

Fig. 2B

46 — 44

30c

Fig. 2C

30d — 44

Fig. 2D

74 — 76

30e

Fig. 2E

74 — 76

30f

Fig. 2F

30g — 78

Fig. 2G

TRANSMIT/RECEIVE
ANTENNA

30c

52

POWER DETECT/
REGULATE
54

RADIO
FREQUENCY
GENERATOR
56

Fig. 3A

60

RADIO
FREQUENCY
GENERATOR
62

RF AMPLIFIER

TRANSMIT
ANTENNA

68

64

66

GAIN ADJUSTMENT

Fig. 3B

30e

RECEIVE
ANTENNA

80

POWER DETECT/
REGULATE
82

OSCILLATOR/
WAFEFORM
GENERATOR
84

PIEZO DEVICE
86

88

90

Fig. 4

EP 1 341 466 B1

*Fig. 5*

*Fig. 6*

Fig. 7

Fig. 8

HALL-EFFECT 32'

120

AMP 122

126

124

VCO

AMP 130

116

SIGNAL METER 126

128 128

34

RECEIVE ANTENNA 140 32''

SELECTABLE NOTCH FILTER 142

RF AMPLIFIER 144

GAIN ADJUSTMENT 146

RECTIFIER/ INTEGRATOR 148

VCO 152

AUDIO AMP 154

SPEAKER 116

ANALOG SIGNAL STRENGTH DISPLAY 150

34''

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

*Fig. 22*

EP 1 341 466 B1

*Fig. 23A*

*Fig. 23B*

*Fig. 23C*

*Fig. 23D*

*1160* *1101*

*1140*

**Fig. 24**

*1160* *1102* *1160*

*1140*

**Fig. 25**

*1160* *1103*

*1140*

**Fig. 26**

*1160* *1104*

*1160* *1140*

**Fig. 27**

*1105*

*1140* *1160*

**Fig. 28**

*1160* *1106*

*1140*

**Fig. 29**

*1160* *1107*

*1140*

**Fig. 30**

*1170* *1108*

*1140*

**Fig. 31**

*1170* *1109*

*1140*

**Fig. 32**

*1170* *1110*

*1140*

**Fig. 33**

Fig. 34

Fig. 35

Fig. 36

Fig. 37

Fig. 38

Fig. 39

A — · — — · 1130a 1124 1121 1120

Fig. 40

A — · — — · 1124 1130a 1130b 1121 1120

Fig. 41

A — · — — · 1130b 1124 1130a 1121 1120 1130c

Fig. 42

1132 1133 1124 1134 1130a 1130b 1121 1120 1130c

Fig. 43

*1133*

ACTUATOR

*1132*

*1157*

*1151*

*1154*

*1152*

*1156*

*1134*

**Fig. 44**

*1133*

ACTUATOR

*1132*

*1130b*

*1134*

D₁

D₂

**Fig. 45**

*1120a*

*1121*

*1130*

*1130*

*1130*

*1124a*

**Fig. 46**

*Fig. 47*

*Fig. 48*

*1120d*

*1124d*

*1130*

*1130*

*1130*

**Fig. 49**

*1120e*

*1121*

*1124e*

*1130*

**Fig. 50**

*1121*

*1130*

*1130*

*1130*

*1124f*

*1120f*

**Fig. 51**

Fig. 52

Fig. 53

Fig. 54

MOTOR

Fig. 55

*Fig. 56*

1562

1121

1550c

1552

1566

1130

1567

1556

1121

1562

1550d

1560

1130b

1130a

1566

1556

1567

*Fig. 57*

1121

1562

1130c

1550e

1560

1130b

1130a

1552

1566

1556

1567

*Fig. 58*

Fig. 59A

Fig. 59B

Fig. 59C

Fig. 59D

EP 1 341 466 B1

*Fig. 60*

*Fig. 61*

*Fig. 62*

*Fig. 63*

Fig. 64

EP 1 341 466 B1

*Fig. 65A*

X, Y, Z = Alignment (Reference) Frame
X, Y, Z = Rotated Stylus or Receiver
        Coordinate Frame

$\psi$ = Azimuth
$\theta$ = Elevation
$\phi$ = Roll

*Fig. 65B*

*Fig. 66*

Fig. 67

Fig. 68

**Fig. 69A**

**Fig. 69B**

Fig. 69C

*Fig. 70*

**Fig. 71**

**Fig. 72**

*Fig. 73*

*Fig. 74*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5630431 A, Taylor
- US 5397329 A, Allen
- US 5170055 A
- US 5246005 A, Carroll
- WO 9838908 A
- WO 9712553 A